# EUROPEAN PATENT SPECIFICATION

(11) **EP 1 660 040 B1**
(45) Date of publication and mention of the grant of the patent: **24.02.2010**
(21) Application number: 04761162.9
(22) Date of filing: 23.08.2004
(51) Int. Cl.: A61K 9/127, A61K 39/00, A61K 31/4172, A61P 35/00, A61P 37/02

(54) **IN VIVO TARGETING OF DENDRITIC CELLS**
IN VIVO TARGETING VON DENDRITISCHEN ZELLEN
CIBLAGE IN VIVO DE CELLULES DENDRITIQUES

(30) Priority: 21.08.2003 AU 2003904513
(43) Date of publication of application: 31.05.2006
(73) Proprietor: Lipotek Pty Ltd., Glen Osmond SA 5064 (AU)
(72) Inventor: ALTIN, Joseph, Holder, ACT 2601 (AU); PARISH, Christopher, Richard, Campbell, ACT 2601 (AU)
(74) Representative: Jones, Elizabeth Louise
(86) International application number: PCT/AU2004/001125
(87) International publication number: WO 2005/018610

(56) References cited:
- WO-A1-00/64471
- VAN BROEKHOVEN CHRISTINA L ET AL: "A novel approach for modifying tumor cell-derived plasma membrane vesicles to contain encapsulated IL-2 and engrafted costimulatory molecules for use in tumor immunotherapy." INTERNATIONAL JOURNAL OF CANCER. JOURNAL INTERNATIONAL DU CANCER 1 MAR 2002, vol. 98, no. 1, 1 March 2002 (2002-03-01), pages 63-72, XP002381951 ISSN: 0020-7136
- VAN BROEKHOVEN C L ET AL: "Engrafting costimulator molecules onto tumor cell surfaces with chelator lipids: a potentially convenient approach in cancer vaccine development." JOURNAL OF IMMUNOLOGY (BALTIMORE, MD. : 1950) 1 MAR 2000, vol. 164, no. 5, 1 March 2000 (2000-03-01), pages 2433-2443, XP002251130 ISSN: 0022-1767
- CHIKH GHANIA G ET AL: "Attaching histidine-tagged peptides and proteins to lipid-based carriers through use of metal-ion-chelating lipids." BIOCHIMICA ET BIOPHYSICA ACTA 23 DEC 2002, vol. 1567, no. 1-2, 23 December 2002 (2002-12-23), pages 204-212, XP002443803 ISSN: 0006-3002
- CHIKH GHANIA ET AL: "Liposomal delivery of CTL epitopes to dendritic cells." BIOSCIENCE REPORTS APR 2002, vol. 22, no. 2, April 2002 (2002-04), pages 339-353, XP008021599 ISSN: 0144-8463
- VAN BROEKHOVEN CHRISTINA L ET AL: "Targeting dendritic cells with antigen-containing liposomes: a highly effective procedure for induction of antitumor immunity and for tumor immunotherapy." CANCER RESEARCH 15 JUN 2004, vol. 64, no. 12, 15 June 2004 (2004-06-15), pages 4357-4365, XP002443804 ISSN: 0008-5472
- ALTIN J G ET AL: "Targeting dendritic cells with antigen-containing liposomes: Antitumour immunity" EXPERT OPINION ON BIOLOGICAL THERAPY 2004 UNITED KINGDOM, vol. 4, no. 11, 2004, pages 1735-1747, XP009045955 ISSN: 1471-2598
- VAN BROEKHOVEN C. ET AL.: 'A novel approach for modifying tumor cell-derived plasma membrane vesicles to contain encapsulated IL-2 and engrafted costimulatory molecules for use in tumor immunotherapy' INT. J. CANCER vol. 98, 2002, pages 63 - 72, XP002381951
- VAN BROEKHOVEN C. ET AL.: 'Engrafting costimulator molecules onto tumor cell surface with chelator lipids: a potentially convenient approach in cancer vaccine development' J. IMMUNOL. vol. 164, 2000, pages 2433 - 2443, XP002251130
- CHIKH G. ET AL.: 'Attaching histidine-tagged peptides and proteins to lipid-based carriers through the use of metal-ion-chelating lipids' BIOCHIM ET BIOPHYS. ACTA vol. 1567, 2002, pages 204 - 212, XP002443803
- CHIKH G. ET AL.: 'Liposomal delivery of CTL epitopes to dendritic cells' BIOSCIENCE REPORTS vol. 22, no. 2, 2002, pages 339 - 353, XP008021599

## Description

### TECHNICAL FIELD

The invention described herein relates generally to the composition of preparations for the targeting of membrane-associated antigen (Ag) to dendritic cells (DCs) in order to modulate immune responses, either for disease prevention or for therapeutic purposes. More particularly, the invention relates to a method of modifying Ag-containing membranes, to enable engraftment and/or incorporation of targeting molecules and immunomodulatory factors, allowing the modified membranes to be targeted to DCs *in vivo* and potently induce, or suppress, immune responses. Even more particularly, the invention relates to a composition that can be used to modify Ag-containing membrane structures, such as liposomes or plasma membrane vesicles (PMVs), to enable the membranes to be targeted effectively to DCs *in vivo,* thereby modulating immunity, and enabling them to be used either as vaccines or vaccine-like agents in immunotherapies to prevent or treat disease in humans and animals.

### BACKGROUND ART

Dendritic cells (DCs) are a rare population of antigen presenting cells (APCs) uniquely capable of stimulating primary immune responses, and a strong interest has developed in their use in cancer immunotherapies.¹ Attempts to harness the capacity of DCs to stimulate potent immune responses have hitherto focused primarily on procedures involving the manipulation of DCs *ex vivo.* This approach often requires that DCs be isolated from a patient, expanded in numbers, loaded with antigen (Ag) (ref's 2-5), and then be re-introduced into the patient. While this procedure is simple in principle, there are difficulties associated with isolation and culture of such a rare cell population. ^{6,7} Clearly, strategies that deliver Ags directly to DCs *in vivo,* and that can elicit an appropriate immune response, have enormous clinical potential.

DCs originate from progenitors in the bone marrow and migrate as immature cells to peripheral tissues where they internalise Ag and undergo a complex maturation process. Ag is internalised via a number of surface receptors, including the complement receptors (e.g., CDllc/CD18) and the endocytic receptors (e.g., DEC-205, DC-SIGN and Toll-like receptors). During Ag acquisition, immature DCs also may receive "danger signals", in the form of pathogen-related molecules such as bacterial cell wall lipopolysaccharide (LPS), or inflammatory stimuli via cytokines such as IFN-γ. DCs then migrate to the secondary lymphoid organs, maturing to become competent APCs.⁸ Receptors such as CD11c/CD18, DEC-205, DC-SIGN and Toll-like receptors play a crucial role in the process of Ag capture and presentation, and are expressed primarily on DCs. It is conceivable, therefore, that these receptors also could be used for targeting Ag directly to DC *in vivo.* Consistent with this notion, fusion proteins composed of Ag and single chain antibodies (ScFvs) to DEC-205 have been shown to target to DCs *in vivo,* inducing T cell activation when co-administered with inflammatory stimulators such as anti-CD40 antibody.^{9,10} In contrast, in the absence of such inflammatory stimulators, antigen targeted to DCs via the ScFv induced T cell unresponsiveness.

Synthetic liposomes have the potential to deliver large quantities of Ags to DCs (Ref. 11), but to date their targeting to specific DC surface molecules has been difficult to achieve in practice.^{12,13} Clearly, an effective strategy that combines the Ag carrying capacity of liposomes and the specificity of molecular recognition to target multiple Ags either with or without "danger signals" directly to DCs *in vivo,* would have enormous potential in simplifying DC immunotherapies, particularly for cancer, infections, and autoimmune diseases.

In International Application No. PCT/AU00/00397 (Publication No. WO 00/64471) there is described a method of modifying biological or synthetic membranes or liposomes for the purposes of altering immunity, or for the targeting of drugs and other agents to a specific cell type or tissue when the modified biological or synthetic membranes or liposomes are administered *in vivo*. Modification of the membranes or liposomes is achieved by the incorporation or attachment of metal chelating groups, thereby allowing engraftment of one or more targeting molecules possessing a metal affinity tag. However, the nature of the immune response induced by targeting Ag to DCs is critically dependent on the presence of specific immunomodulatory factors such as cytokines or "danger" signals, and there is no disclosure or suggestion in PCT/AU00/00397 of the membrane modification that is required, or the immunomodulatory factors that are needed, to elicit an appropriate immune response *in vivo.*

### SUMMARY OF THE INVENTION

An object of the invention the subject of this application, is to provide a composition for the *in vivo* targeting to DCs, of Ag-containing liposomes and PMV, by modifying the said membranes through incorporation of an appropriate immunomodulatory factor, or "danger signal", and the engraftment of a ligand, that can target the modified membranes to receptors on the surface of DCs, and hence elicit an appropriate immune response. The composition can be used as vaccines or in immunotherapies, either to potentiate immunity for preventing or treating diseases such as various cancers and infections, or to suppress immunity to a specific self Ag in a way that can be used to treat or prevent transplant rejection, or the effects of autoimmune diseases such as type I diabetes, rheumatoid arthritis, systemic lupus erythematosus and multiple sclerosis.

Further objects of the invention are to provide a process for preparing suitable compositions, and methods of treatment utilising the compositions.

According to a first embodiment of the invention, there is provided a composition for modulating immunity by the *in vivo* targeting of an antigen to dendritic cells, the composition comprising:
a preparation of antigen-containing membrane vesicles or antigen-containing liposomes having on the surface thereof a plurality of metal chelating groups; and
a ligand for a receptor on said dendritic cells, said ligand being linked to a said metal chelating group via a metal affinity tag on said ligand; wherein,
said antigen-containing vesicles or liposomes include an immunomodulatory factor.

According to a second embodiment of the invention, there is provided a process for preparing a composition for modulating an immune response by the *in vivo* targeting of an antigen to dendritic cells, the process comprising the steps of:
i) preparing antigen-containing membrane vesicles or antigen-containing liposomes;
ii) modifying said antigen-containing membrane vesicles or antigen-containing liposomes by the incorporation of at least one immunomodulatory factor;
iii) further modifying said antigen-containing membrane vesicles or antigen-containing liposomes by the incorporation of amphiphilic molecules, wherein said amphiphilic molecules include a chelator group which lies on the surface of said antigen-containing membrane vesicles or antigen-containing liposomes when incorporated therein; and
iv) contacting the product of step (iii) with a ligand for a receptor on said dendritic cells, wherein said ligand includes a metal affinity tag for binding to said chelator group.

According to a third embodiment of the invention, there is provided a method of modulating an immune response in a subject, the method comprising administering to said subject a composition according to the first embodiment.

According to a fourth embodiment of the invention, there is provided a method of preventing or treating a tumour in a subject, the method comprising administering to the subject a composition according to the first embodiment, wherein said antigen included in said antigen-containing membrane vesicles or antigen-containing liposomes is a tumour antigen.

According to a fifth embodiment of the invention, there is provided a method of preventing or treating an infection in a subject, the method comprising administering to the subject a composition according to the first embodiment, wherein said antigen included in said antigen-containing membrane vesicles or antigen-containing liposomes is an antigen from an agent causing the infection.

According to a sixth embodiment of the invention, there is provided use of a composition according to the first embodiment in the preparation of a medicament for modulating an immune response in a subject.

According to a seventh embodiment of the invention, there is provided use of a composition according to the first embodiment in the preparation of a medicament for preventing or treating a tumour in a subject.

According to an eighth embodiment of the invention, there is provided use of a composition according to the first embodiment in the preparation of a medicament for preventing or treating an infection in a subject.

Other embodiments of the invention will become apparent from a reading of the following detailed description of the invention, in which description there will be reference to the accompanying drawings briefly described hereafter.

### BRIEF DESCRIPTION OF THE DRAWINGS

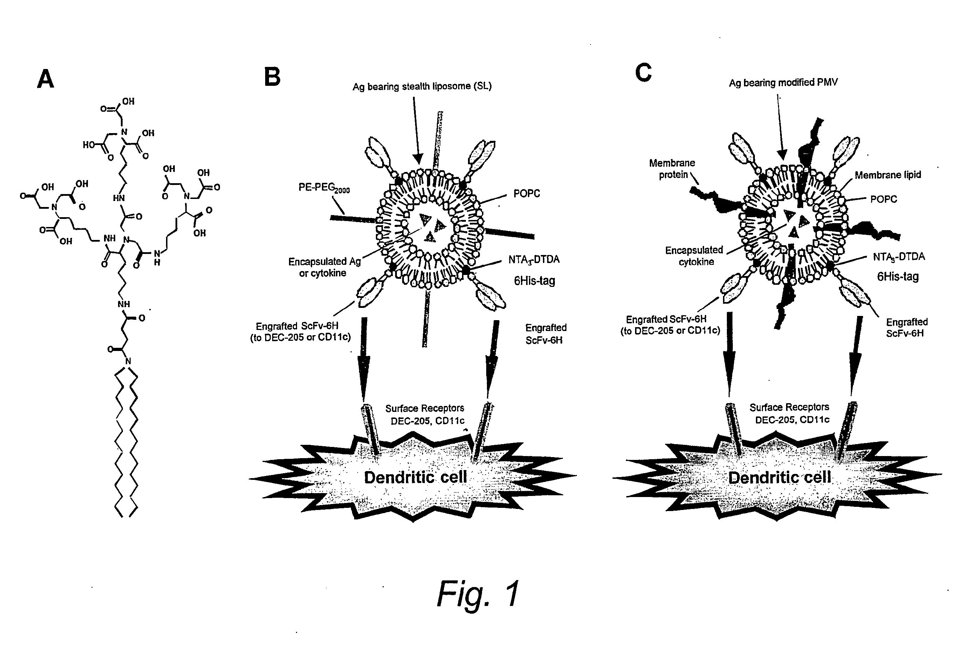

Figure 1 shows the structure of the novel chelator lipid (NTA)₃-DTDA. Figure 1B is a schematic representation of the (NTA)₃-DTDA lipid incorporated in antigen (Ag) containing stealth liposomes (SL) composed of palmitoyl-oleoyl-phosphatidylcholine (POPC) and phosphatidyl-ethanolamine-(polyethylene) glycol₂₀₀₀ (PE-PEG₂₀₀₀). Figure 1C is similarly a schematic representation of liposomes of similar composition to those of Figure 1B but without PE-PEG₂₀₀₀ can be fused with antigen-bearing tumour cell-derived plasma membrane vesicles (PMV). In both instances, SL (B) and modified PMV (C), the lipid tracer PC-BODIPY (not shown) can also be included to facilitate tracking of either the liposomes or the modified PMV. The (NTA)₃-DTDA permits the engraftment of histidine-tagged ScFv Abs against DEC-205 and CD11c onto the liposome or modified PMV surface, and consequently, the targeting of these to surface markers such as DEC-205 and CD11c on DCs.

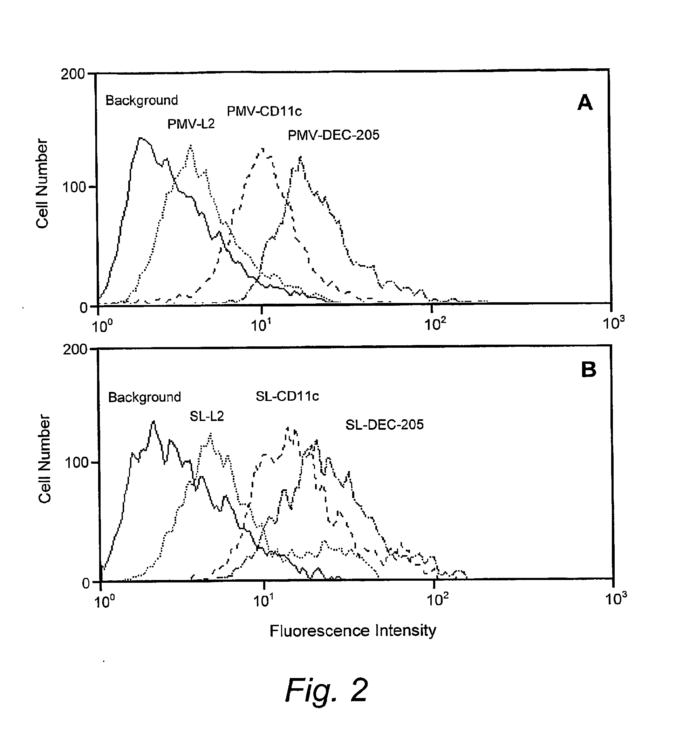

Figure 2 shows that PMV and SL engrafted with CD11c-ScFv and DEC-205-ScFv bind to DCs. With regard to Figure 2A, PMV derived from B16-OVA cells were fused with liposomes composed of POPC, (NTA)₃-DTDA, and PC-BODIPY. The PMV were then engrafted with a control peptide (PMV-L2), CD11c-ScFv (PMV-CD11c), or DEC-205-ScFv (PMV-DEC-205), before being incubated with LTC-DC and cell bound BODIPY-fluorescence quantified by flow cytometry. Figure 2B shows the binding to LTC-DC of similarly-engrafted SL composed of POPC, (NTA)₃-DTDA, PE-PEG₂₀₀₀ and PC-BODIPY. Each profile is representative of that obtained from three separate experiments.

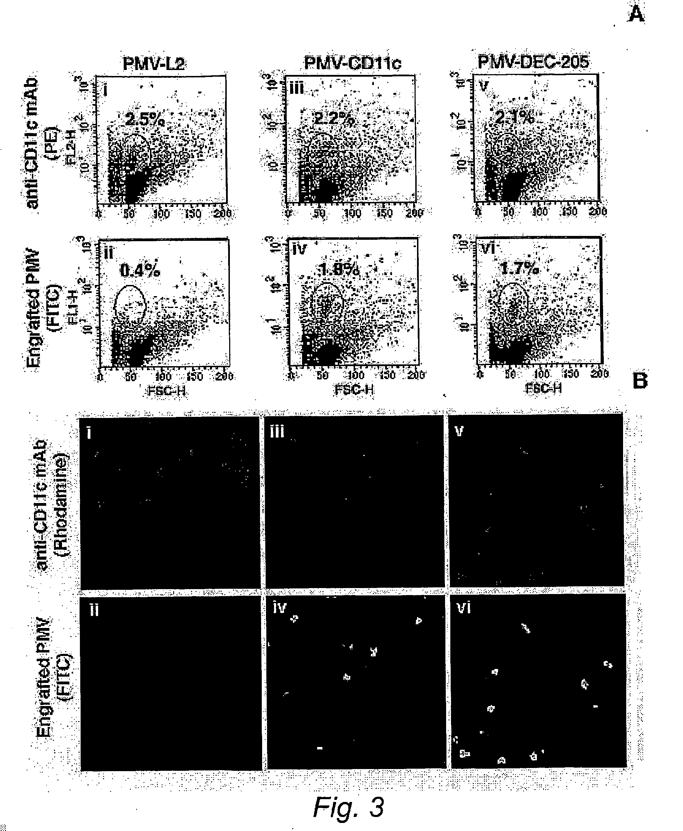

Figure 3 shows that ScFv-engrafted PMV target DCs in draining lymph node. Mice were injected in the hind footpad with fluorescein-labelled PMV that had been engrafted with either control protein (PMV-L2), or with ScFv to CD11c (PMV-CD11c) and to DEC-205 (PMV-DEC-205). **A.** After the injection the draining popliteal lymph nodes were removed for staining of isolated lymph node cells with a biotinylated anti-CD11c mAb and PE-streptavidin. Flow cytometry dot plots show double staining depicting PE-fluorescence (panels i, iii and v), and corresponding FITC-fluorescence (panels ii, iv and vi) of lymph node cells, as indicated. **B.** Results of similar experiments in which sections of lymph node were stained with a biotinylated anti-CD11c mAb and streptavidin-Rhodamine to identify DCs with the fluorescence images of corresponding fields depicting Rhodamine-fluorescence (images i, iii and v), and PMV fluorescein fluorescence (images ii, iv and vi).

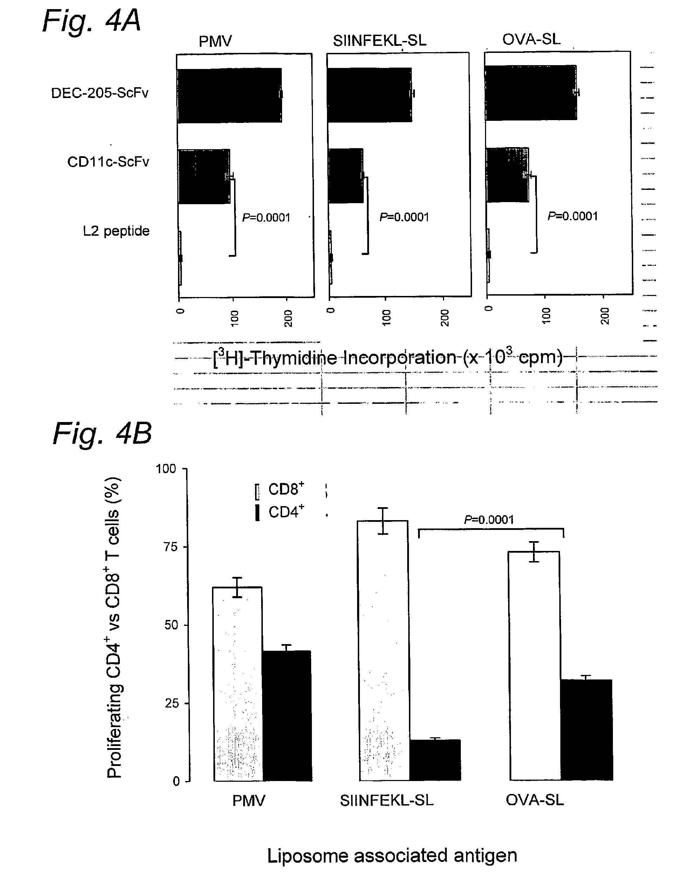

Figure 4 shows that targeting engrafted PMV and SL to DCs stimulates T cell proliferation. **A.** Syngeneic C57BL6 splenic T cells were incubated with unpulsed DCs, or with DC which had been pulsed with B16-OVA PMV engrafted with L2, CD11c-ScFv, or DEC-205-ScFv (left panel); SL bearing SIINFEKL-6H engrafted with L2, CD11c-ScFv or DEC-205-ScFv (middle panel); and OVA-containing SL engrafted with L2, CD11c-ScFv or DEC-205-ScFv (right panel). The cells were cultured for 4 days before assessing [³H]-thymidine incorporation; results are cpm ± SEM. **B**. Stimulation of CD4⁺ and CD8⁺ T cell proliferation. Syngeneic C57BL6 splenic T cells labelled with CFSE were incubated with DCs pulsed with PMV engrafted with DEC-205-ScFv (PMV), SL engrafted with DEC-205-ScFv and SIINFEKL-6H (SIINFEKL-SL), and OVA-containing SL engrafted with DEC-205-ScFv (OVA-SL). The cells were cultured for 4 days and the relative proportion of proliferating CD4⁺ and CD8⁺ T cells, based on CFSE dilution, assessed by flow cytometry.

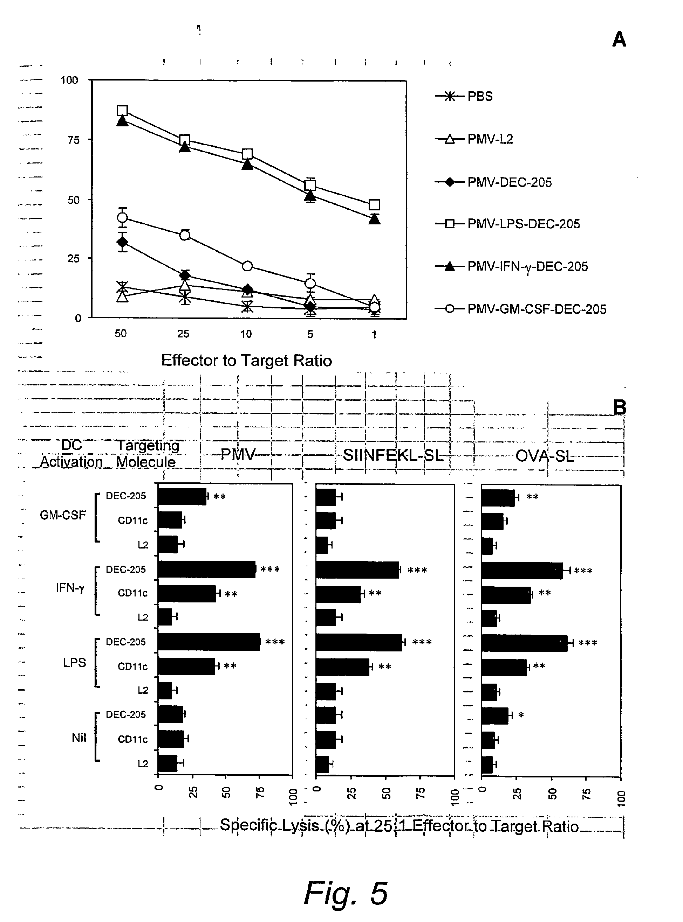

Figure 5 comprises the results of the vaccination of mice with PMV and SL and shows stimulation of CTL activity against tumour cells. **A**. CTL activity of splenocytes stimulated for 4 days with γ-irradiated B16-OVA cells and derived from mice injected i.v. with PBS alone (PBS), B16-OVA PMV engrafted with L2 peptide (PMV-L2), PMV engrafted with DEC-205-ScFv alone (PMV-DEC-205), or in combination with LPS (PMV-LPS-DEC-205), IFN-γ (PMV-IFN-γ-DEC-205), or GM-CSF (PMV-GM-CSF-DEC-205). **B**. The CTL activity of splenocytes (25:1 E:T ratio) from mice following immunisation with PMV, SIINFEKL-containing SL, and OVA-containing SL, each engrafted with L2, CD11cScFv or DEC-205-ScFv, as indicated. Results for conditions in which LPS, IFN-γ and GM-CSF were incorporated with the engrafted PMV and SL, as indicated, also are shown. Asterisks indicate that CTL activity is significantly higher (n=6. *, *P*<0.05; **, *P*<0.01 and **, *P*<0.001) than mice immunised with a corresponding Ag preparation engrafted with L2 peptide. In panels A and B specific lysis at the indicated E:T ratios, was assessed in a standard ⁵¹Cr release assay. Results are expressed as the percentage specific lysis ± SEM.

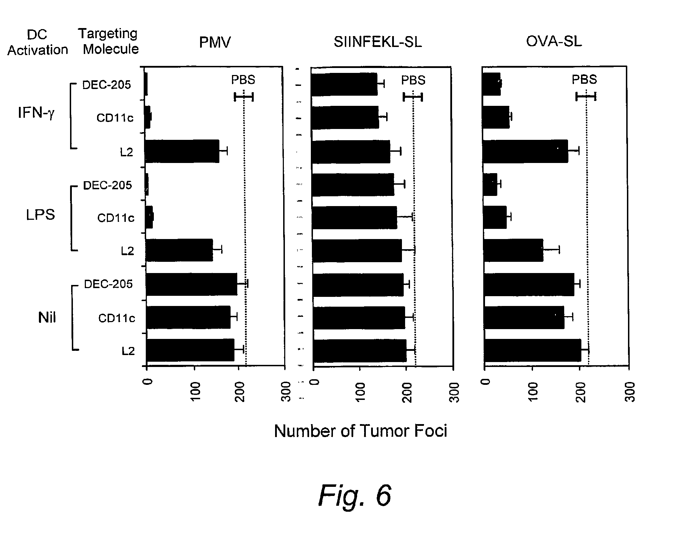

Figure 6 shows that vaccination with modified PMV and SL elicits tumour immunity. Separate groups of syngeneic C57BL6 mice were immunised (three times at weekly intervals) with PMV engrafted with L2, CD11c-ScFv, or DEC-205-ScFv; SL engrafted with SIINFEKL-6H and L2, CD11c-ScF or DEC-205-ScFv; and OVA-containing SL engrafted with L2, CD11c-ScFv, or DEC-205-ScFv, with each vaccine preparation being injected alone or in combination with LPS or IFN-γ, as indicated. Mice were challenged i.v. with B16-OVA cells, and after 16 days the lungs were removed and examined for lung metastases. Results show the mean number of tumour foci for each group of mice ±SEM. The dotted line refers to the number of tumour metastases in control mice that were injected with PBS.

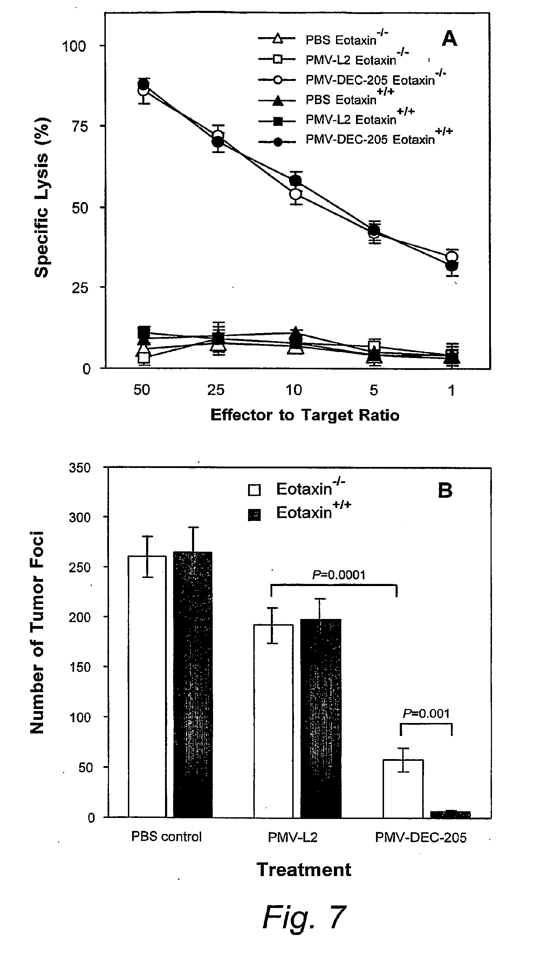

Figure 7 depicts anti-tumour responses in eotaxin knockout mice. **A**. Syngeneic C57BL6 mice (Eotaxin^{+/+}) or eotaxin knockout mice (Eotaxin^{-/-}) on a C57BL6 background, were immunised with PBS, or with IFN-γ-containing PMV engrafted with either L2 (PMV-L2) or DEC-205-ScFV (PMV-DEC-205), as indicated. Splenocytes were isolated from the mice, and co-cultured with γ-irradiated native B16-OVA cells. Specific lysis at the indicated E:T ratios, was assessed in a standard ⁵¹Cr release assay. Results are expressed as the percentage specific lysis ±SEM. **B**. Mice were immunised as above and then challenged i.v. with B16-OVA cells, with the lungs being removed and examined after 16 days for tumour metastases. Results show the mean number of tumour foci for each group of mice ±SEAM.

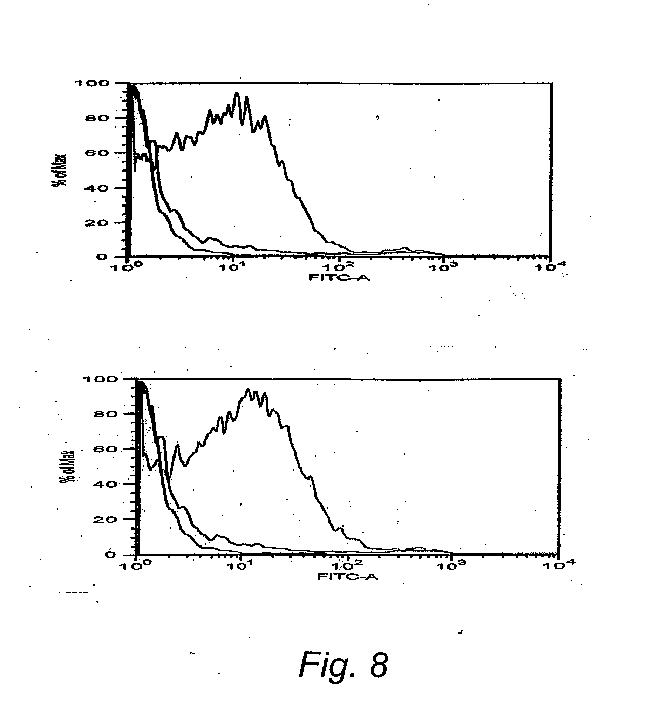

Figure 8 shows that membrane vesicles of BCG mycobacteria engrafted with CD11c-ScFv and DEC-205-ScFv bind to DCs. Ni-(NTA)₃-DTDA was combined with PMV derived from BCG mycobacteria and labelled with 6-(fluoresein-5(and-6)-carboxamido)hexanoic acid succinimidyl ester. The PMV were then engrafted with a control peptide (BCG-Lipo+L2), CD11c-ScFv (BCG-Lipo+CD11c), or DEC-205-ScFv (BCG-Lipo+DEC205), before being incubated with JAWS-11 DC after which cell bound fluorescence was quantified by flow cytometry. The upper panel shows the results for BCG-Lipo+CD11c while the lower panel comprises the results for BCG-Lipo+DEC205. In each panel, the left-hand trace is for the JAWS-11 cells alone, the middle trace is for the BCG-Lipo+L2 control, while the right-hand trace is for BCG-Lipo+CD11c or BCG-Lipo+DEC205.

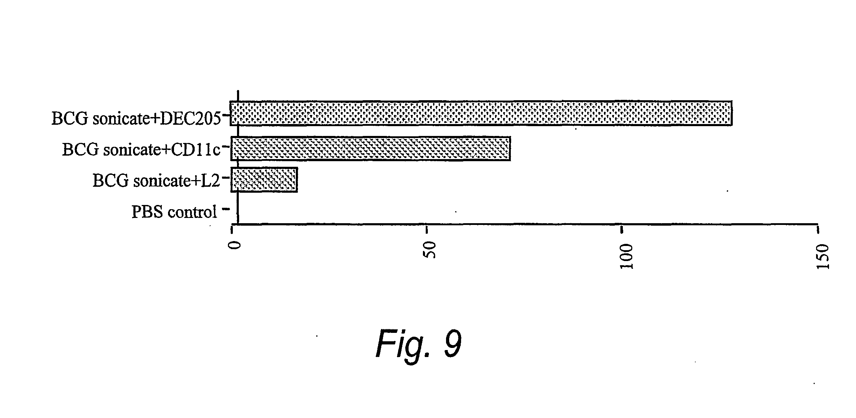

Figure 9 depicts the results of an Elispot analysis of splenic T cells from C57/BL6 mice that had been vaccinated intravenously with engrafted BCG preparations. The engraftments were: L2 peptide as a control (BCG sonicate+L2); CD11c-ScFv (BCG sonicate+CD11c); or DEC-205-ScFv (BCG sonicate+DEC205). Control mice were vaccinated with the PBS used as a carrier for the preparations.

### DETAILED DESCRIPTION OF THE INVENTION

The following abbreviations are used herein:

| | |
|---|---|
| Ag | antigen |
| APC | antigen presenting cell |
| CTL | cytotoxic T lymphocyte |
| DC | dendritic cell |
| IFN-γ | interferon-γ |
| LPS | lipopolysaccharide |
| (NTA)₃-DTDA | tri(nitrilotriacetic acid) ditetradecylamine |
| OVA | ovalbumin |
| PMV | plasma membrane vesicle |
| ScFv | single chain antibody fragment |
| SL | stealth liposome |

The term "antigen" is used herein to denote any molecule which can be taken up, internalised and processed by DCs, for presentation to the immune system.

The term "ligand" is used herein to denote any molecule which can specifically bind *in vivo* to markers/receptors on the surface of DCs. The term includes whole antibodies, and antibody fragments such as ScFvs and domain antibodies.

The term "immunomodulatory factor" is used herein to denote any "danger signal", cytokine or molecule that can modulate the course or outcome of an immune response.

The term "receptor" is used herein to denote a receptor molecule on the surface of a DC, and is the entity on the DC surface with which a liposome- or membrane vesicle-engrafted ligand can interact.

The term "tumour" is used herein to denote benign and malignant solid tumours as well as solid and non-solid cancers.

With regard to the first embodiment defined above, the antigen-containing membrane vesicles are typically PMVs but can be formed from any biological membrane or biological structure. The PMVs are advantageously tumour-derived PMVs. The PMVs can also be lymphocyte-derived PMVs or leucocyte-derived PMVs. The PMVs can be furthermore membranous preparations of bacteria, protozoa, viruses or fungi. With regard to the antigen-containing liposomes, these include stealth liposomes (SLs) which can be produced from different mixtures of lipids. Such vesicles and liposomes can be prepared as described in references 14, 16, 17 and 28.

The Ag of the compositions can be any Ag, or DNA encoding an Ag, against which an immune response is desired. A composition can comprise a plurality of different antigens which may be from the same or a different source. That is, a composition comprising tumour antigens may include antigens from different tumours.

The metal chelating groups on the surface of the vesicles and liposomes exist as headgroups of amphiphilic molecules present within the phospholipids and/or lipids comprising the vesicles and liposomes. The amphiphilic molecule is advantageously nitrilotriacetic acid ditetradecylamine (NTA-DTDA) or nitrilotriacetic acid phosphatidylethanolamine (PE-NTA), but compositions can include any molecule containing any metal binding or chelating moiety that can be incorporated into lipid membranes. Compositions can furthermore comprise mixtures of amphiphilic molecules.

As will be explained in greater detail below, a preferred amphiphilic molecule is (NTA)₃-DTDA (tri(nitrilotriacetic acid) ditetradecylamine). The related molecule NTA-DTDA, together with other amphiphilic molecules and vesicles and liposomes containing the same, are described in greater detail in International Application No. PCT/AU00/00397 (Publication No. WO 00/64471).

The ligand linked to the metal chelating groups on the membrane vesicles and liposomes can be any metal-affinity tagged molecule that can bind specifically to any DC surface marker. A preferred metal-affinity tag is hexahistidine. In examples below, hexahistidine-tagged forms of ScFv against the DC surface molecules CD11c and DEC-205 (CD205) are used. Other examples include any histidine-tagged ligand such as an antibody or antibody fragment that can bind to DC surface markers such as DC-SIGN (CD209), CD206 and CD207.

Compositions can include a plurality of ligands for different markers/receptors on DCs. For example, a composition can comprise as ligands an ScFv against DEC-205 in combination with an ScFv against DC-SIGN.

As indicated above, the metal affinity tag of a ligand is typically a hexahistidine moiety covalently linked at a convenient site on the ligand. For example, the hexahistidine can be linked to a protein antigen at the N- or C-terminal thereof. Other metal affinity tags include any moiety or amino acid sequence that can chelate metals and that can be covalently attached to a convenient site on the ligand.

The immunomodulatory factors of compositions according to the first embodiment include compounds or molecules that can enhance or modify the response of DCs to antigens. Such compounds include "danger signals" (e.g., bacterial lipopolysaccharide), cytokines (e.g., interferon-γ, interleukin-2, interleukin-4, interleukin-10, interleukin-12 and transforming growth factor-β), as well as chemokine, hormonal and growth factor-like molecules, or DNA encoding such molecules. A composition can include more than one immunomodulatory factor.

Concerning the second embodiment of the invention, suitable processes for the preparation of membrane vesicles or liposomes with ligand entrapped thereon are described in the international application (No. PCT/AU00/00397) referred to above.

With regard to step (i) of the second embodiment process, the membrane vesicles are typically PMVs but can be formed from any biological membrane or biological structure. The liposomes include SLs. The Ag of the membrane vesicles and liposomes can be protein, glycoprotein, peptide or polysaccharide, or DNA encoding an antigen, or combinations thereof, to be delivered to the DCs.

In step (ii) of the second embodiment process, as with the first embodiment composition, the immunomodulatory factor can be a "danger signal" (e.g., a bacterial lipopolysaccharide), a cytokine (e.g., interferon-γ, interleukin-2, interleukin-4, interleukin-10, interleukin-12 and transforming growth factor-β), or DNA encoding such factors.

The immune response modulation of the method according to the third embodiment has application in the prevention or treatment of conditions which include transplant rejection, or the effects of autoimmune diseases such as type I diabetes, rheumatoid arthritis, systemic lupus erythematosus and multiple sclerosis. In the case of transplant patients, this involves the administration of PMVs from donor leukocytes that are targeted to the DCs of the transplant recipient. The immunomodulatory factor in this instance can be, for example, a cytokine such as interleukin-10 or transforming growth factor-β. However, the immunomodulatory factor can be any molecule that has the ability to generate tolerogenic DCs.

The method of the fourth embodiment can be used in the treatment of any tumour including, but not limited to, melanoma, and cancers of the prostate, bowel, breast and lung. The method can also be used in the treatment of leukaemia and lymphomas. The method can be used to treat tumours in any mammalian animal but is particularly suited for treating tumours in humans.

The amount of modified Ag-containing membrane vesicles or liposomes to be delivered to a subject and the administration regime can be established by the clinician after assessment of the subject in the light of the tumour under treatment.

Those of skill in the art will immediately recognise that the method according to the fourth embodiment provides an effective alternative to the *ex vivo* manipulation of DCs for use in cancer immunotherapy.

With regard to the fifth embodiment, the method can be used to prevent or treat any infection including infections caused by bacteria, mycobacteria, viruses and fungi in order to enhance immunity to the agent responsible for the infection and/or for use in the treatment of an infection. In a similar fashion to the example given above for the prevention of transplant rejection, all that is required to provide an efficacious method is to prepare PMVs or liposomes that include at least one antigen from the infectious agent. That antigen can be, for example, envelope proteins of viruses (e.g., HIV, hepatitis B and C) or cell wall components of bacteria (e.g., Mycobacteria), fungi (e.g., Candida) and protozoa (e.g., malaria).

Administration of compositions to a subject in accordance with the third to fifth embodiments of the invention can be by any of the methods known to those of skill in the art. Compositions are typically administered intravenously or subcutaneously.

The subject of the methods of the third to fifth embodiments is typically a mammalian subject. The methods are particular suited for use with a human subject.

Those of skill in the art will appreciate that a medicament according to the sixth to eighth embodiment of the invention will also include at least a carrier for the composition. The carrier can be any solution with which PMVs and liposomes are compatible. Typical carriers are saline and buffered saline such as PBS.

Medicaments can include further active agents consistent with the intended use of the medicament. For example, a medicament according to the seventh embodiment can include other anti-tumour agents while a medicament according to the eighth embodiment can include other agents with anti-bacterial, anti-protozoan, anti-viral or anti-fungal activity as appropriate for the target infection. Such additional agents will be known to those of skill in the art.

### Prototype Studies

In a prototype study, the inventors have found that the chelator-lipid (NTA)₃-DTDA can be used to anchor His-tagged ScFv onto either tumour-derived plasma membrane vesicles (PMV) or onto tumour antigen-containing stealth liposomes for the targeting of DCs. Targeting of Ag directly to DCs in this way elicited a strong anti-tumour response.

Liposomes have been hailed as having high therapeutic potential, but their use has been hampered by a lack of a simple method for attachment of targeting molecules.¹³ The novel chelator-lipid, (NTA)₃-DTDA (Fig. 1A), when incorporated into either SLs or into tumour cell-derived PMV (B16-OVA), enables the stable engraftment of hexa-histidine-tagged ScFv that target surface molecules on DCs (Fig. 1B and Fig. 1C). PMV and SLs engrafted with ScFv specific for the DC markers CD11c and DEC-205 bind specifically to DC *in vitro* and, based on flow cytometry and confocal microscopy studies, can target associated Ags directly to DCs *in vivo* (Fig's 2 and 3).

Initially, the ability of engrafted PMV and SL to stimulate functional responses in assays of DC-initiated Ag presentation was examined. Our studies show that ScFv-engrafted PMV and Ag-containing SL are significantly more effective than control PMV and SLs at inducing DCs to stimulate T cell proliferation (Fig. 4A). With PMV, proliferation was stimulated in both CD4⁺ and CD8⁺ T cells. PMV have the potential to stimulate responses mediated by all possible T cell clones reactive to epitopes present in the tumour cell vesicles. Similarly, ScFv-engrafted SL containing the OVA protein may stimulate both OVA-specific CD4⁺ and CD8⁺ T cells; but SL containing SIINFEKL, the immunodominant CTL epitope in OVA, would be expected to generate only CD8⁺ T cell responses. Consistent with this, our data show that DCs targeted by engrafted PMV generate approximately equal proportions of CD4⁺ and CD8⁺ T cells, whereas DCs targeted by SL containing SIINFEKL, and to a lesser extent those containing OVA, generate predominantly CD8⁺ T cells (Fig. 4B).

Evidence suggests that "danger" signals are important in the maturation and migration of DCs after Ag exposure, and can avoid induction of tolerance to the presented Ag.^{9,10,18} Notably, "danger" signals were not required in the *in vivo* Ag presentation assays (Fig. 4), presumably since the DCs are "perturbed" or activated during their isolation. LPS and cytokines like GM-CSF and IFN-γ are known to influence the ability of DCs to take up Ag and to mature.^{8,19-21} For animal studies therefore, we incorporated LPS, IFN-γ or GM-CSF, within PMV and SL, thereby providing the means to simultaneously deliver both Ag and a danger signal to DCs.

An examination of the ability of ScFv-engrafted PMV and SL containing Ag to induce DCs to initiate CTL responses revealed that, compared to control cells, T cells from animals immunised with ScFv-engrafted PMV or Ag bearing SL exhibit an increased ability, following *in vitro* restimulation, to lyse B16-OVA target cells *in vitro* (Fig. 5). Importantly, the results show that *in vivo* priming for cytolytic activity is dependent on the presence of "danger" signals, with LPS and IFN-γ stimulating the greatest response (Fig. 5). Both the xenogeneic OVA protein, and a hexahistidine-tagged form of SIINFEKL, could be associated with SLs for targeting via the engrafted ScFv. Ag presentation and CTL assays thus demonstrate that targeting ScFv-engrafted PMV and Ag bearing SLs to DCs in this way can be effective in stimulating anti-tumour responses, and lighlights the importance of "danger" signals in the induction of immune responses (Fig's 4 & 5). Moreover, the finding that ScFv-engrafted SL containing SIINFEKL-6H can induce a significant cytotoxic response, demonstrates that the approach using (NTA)₃-DTDA-containing SLs may be an effective strategy for targeting any His-tagged peptide Ag to DCs *in vivo.*

A finding of paramount importance in this work was our observation that syngeneic animals immunised with CD11c-ScFv- and DEC-205-ScFv-engrafted PMV had a significantly lower number of tumour metastases in the lungs compared to controls, after challenge with the B16-OVA melanoma. Similarly, syngeneic animals immunised with ScFv-engrafted SL containing OVA and either LPS or IFN-γ had a lower number of metastases (Fig. 6). The results further show that tumour immunity was completely dependent on the presence of the "danger" signals, LPS and IFN-γ (Figs 5 and 6). The immunisation of mice with CD11c-ScFv- and DEC-205-ScFv-engrafted PMV and Ag bering SL, therefore, target the associated Ag(s) to DCs, which then process and present the Ags to T cells inducing Ag-specific T cell activation, and elicit a strong inhibition in the growth and metastasis of the B16-OVA tumour *in vivo.* A further significant finding was the fact that, unlike control mice which all developed severe lung metastases, mice that had been vaccinated with DEC-205-ScFv-engrafted PMV containing IFN-γ after challenge with B16-OVA tumour cells subsequently did not show any signs of tumour development, indicating that the DC targeting vaccine has therapeutic activity.

A particular intriguing aspect of this study is that the apparent generation of CTL activity against the B16-OVA melanoma was not associated with tumour protection. This point is particularly evident with the SIINFEKL-SL vaccine that would be expected to generate only a CD8⁺ CTL response against OVA produced by the B16-OVA tumour cells. Despite the vaccine inducing a strong *in vitro* recall CTL response against B16-OVA tumour cells, no *in vivo* protection against the tumour was afforded by the immunisation. It is known that the B16-OVA melanoma line expresses very low levels of MHC class I, and consequently, is resistant to CTL lysis unless high avidity CTLs are used.¹⁴ The fact that splenocytes from mice immunised with DC targeting preparations of PMV or SL could lyse B16-OVA tumour cells after restimulation with tumour cells *in vitro* implies that high avidity CTLs can be generated against this tumour cell line. Presumably, such CTLs are either not generated, or are not effective *in vivo.* In fact, previous studies indicate that CD4⁺ rather than CD8⁺ T cells are effective against B16-OVA metastases, with CD4⁺ T cells with a cytokine profile characteristic of T helper 2 (Th2) cells being particularly effective.¹⁴ Furthermore, eotaxin dependent recruitment of eosinophils into the tumours was essential for tumour regression to be observed.¹⁴ To explore a possible role of CD4⁺ T cells-mediated eosinophil recruitment in the anti-tumour effects observed in this study, eotaxin knockout mice were immunised with ScFv-engrafted PMV. Our results show that compared to controls, eotaxin knockout mice exhibit a markedly reduced ability to inhibit the growth and metastasis of the B16-OVA tumour (Fig. 7A). Eotaxin is a potent eosinophil chemokine and therefore, the findings are consistent with the recruitment of eosinophils into the tumour constituting an important component of the anti-tumour response.

The modified PMV and SL system described herein offers a number of advantages over current strategies using DCs for tumour immunotherapy. Firstly, the system can deliver Ags directly to DCs *in vivo,* thus eliminating the need to isolate DCs from patients and to manipulate the cells *ex vivo* for use in immunotherapies. Secondly, a targeted or active liposome-mediated delivery of Ag to DC has the potential to deliver more Ag, and/or several different Ags, simultaneously, potentially stimulating a more effective immune response. The same approach could potentially deliver to DCs any Ag or immunostimulatory agent, such as "danger" signals, RNA, DNA, and cytokines, or combinations thereof, which cannot be easily achieved using Ags fused to DC targeting proteins.^{9,10} Thirdly, the approach is versatile and would be convenient to use clinically since potentially any DC targeting protein(s) possessing a histidine tag can be engrafted onto the modified PMV or SL to deliver specific tumour Ags or other agents to enhance tumour immunity in patients.

Having broadly described the invention and particular application thereof in the foregoing prototype studies, specific examples will now be given after detailing the materials and methods used therein. It will be understood by those of skill in the art that these examples are for illustrative purposes only and do not in any way limit the scope of the invention.

### Materials and Methods

### Reagents

[³H]-Thymidine and ⁵¹Cr (Na⁵¹CrO₄) were obtained from Amersham (Buckingham-shire, United Kingdom). Palmitoyl-oleoyl-phosphatidyl-choline (POPC), OVA (Grade II, purified by FPLC), LPS (from *Escherichia coli* serotype 0111:B4), Isopaque, Ficoll and β-mercaptoethanol were supplied by Sigma-Aldrich (Castle Hill, New South Wales, Australia). Phosphotidylethanolamine-polyethylene glycol-2000 (PE-PEG₂₀₀₀) was obtained from Avanti Polar Lipids Inc. (Alabaster). 2-(4,4-difluoro-5octyl-4-bora-3a,4a-diaza-*s*-indacene-3-pentanoyl)-1-hexadecanoyl-*sn*-glycero 3-phosphocholine (PC-BODIPY) and 5-(and-6)-carboxyfluorescein diacetate, succinimidyl ester, mixed isomers (CFSE) were purchased from Molecular Probes (Eugene, Oregon). The chelator-lipid (NTA)₃-DTDA, consisting of three nitrilotriacetic acid (NTA) head groups covalently linked to two ditetradecylamine (DTDA) chains was synthesized essentially as described,²⁷ but with additional steps to covalently couple a NTA group onto each carboxyl group of the NTA-DTDA, to produce (NTA)₃-DTDA. NiSO₄ was used for all additions of Ni²⁺ to buffers.

### Monoclonal antibodies and proteins

Murine CD56 (clone 42.18, rat IgG2a) mAb was from the 6^{th} Human NK Cell Workshop and the murine CD3 mAb (clone 145-2C11, Armenian hamster IgG) was purchased from PharMingen (San Diego, California). Recombinant murine IFN-γ and GM-CSF were supplied by PeproTech Inc (Rockey Hill, New Jersey). Recombinant ScFv antibodies N418 (anti-CD11c) and NLDC145 (anti-DEC-205), each with a hexahistidine (6H) tag at the carboxy terminal and denoted CD11c-ScFv and DEC-205-ScFv, respectively, were produced using the baculovirus protein expression system and purified as described.^{16,28} Peptides were synthesised by the Biomolecular Resource Facility, John Curtin School of Medical Research (JCSMR), ANU, Canberra. The L2 peptide (GHHPHGHHPH), a sequence of ten amino acids found in the plasma protein histidine-rich glycoprotein, was used routinely to engraft control PMV and SL since it binds to Ni-(NTA)₃-DTDA with high avidity and can block its non-specific binding to cells. The peptide SIINFEKL-6H, representing the immunodominant CTL epitope of OVA in H-2^{b} mice (OVA residues 257-264), with hexahistidine tag attached was used for peptide Ag delivery to DCs.

### Mice and cell lines

Female or male C57BL6 mice (H-2^{b}) 6-8 weeks of age were supplied by the Animal Breeding Establishment, (JCSMR, ANU), and C57BL6 eotaxin knockout mice (H-2^{b}) (eotaxin^{-/-}) were a gift from Dr Paul Foster, Division of Biochemistry and Molecular Biology (JCSMR), and were used to obtain lymphoid cells for *in vitro* assays, and in tumour growth studies *in vivo.* The highly metastatic murine B16-OVA melanoma [C57BL6 (H-2^{b})], an OVA-secreting tumour cell line was cultured at 37°C in an atmosphere of 5% CO₂ in RPMI 1640 medium (Gibco-BRL, Invitrogen, Melbourne, Australia) containing 10% fetal calf serum (FCS, Trace Biosciences, Noble Park, Victoria, Australia) and 0.5 mg/mL Geneticin (Invitrogen). Murine Foetal Skin Dendritic Cells (FSDC) [C57BL6-DBA/2J F1 (H-2^{b}/^{d})] were cultured in the same medium but without Geneticin. Murine Long Term Culture Dendritic Cells (LTC-DC) [B10.A(2R) (H-2^{k/b})], isolated and cultured as described,²⁹ were a gift from Dr H. O'Neill (School of Biochemistry and Molecular Biology, ANU).

### Isolation of dendritic cells and T cells

Murine DC and T cells were isolated from the spleens of C57BL/6 mice. Briefly, splenocytes were isolated by digestion with Collagenase IV (Boerhringer Mannheim), followed by isolation of low density splenocytes by density gradient centrifugation using an Isopaque-Ficoll gradient. DCs were isolated by plastic adherence as described³¹ and then suspended in complete RPMI 1640 growth medium containing 10% FCS, 5 x 10⁻⁵ M β-mercaptoethanol, 100 IU/ml penicillin, 100 µg/ml neomycin, and 10 mM HEPES. For isolation of T cells, the spleens were dissociated into single cell suspensions, and after removing red cells by hypotonic lysis, the T cells were isolated using a nylon wool column.³²

### Plasma membrane vesicles and stealth liposomes

PMV from cultured cells were prepared by sucrose gradient centrifugation,³⁰ and modified essentially as outlined.^{16,17} Liposomes used to modify PMV were prepared as follows: ethanolic solutions of POPC, (NTA)₃-DTDA, LPS and PC-BODIPY (molar ratio 94:2:2:2); or POPC, (NTA)₃-DTDA and PC-BODIPY (molar ratio 96:2:2), were mixed, dried under a stream of N₂, then rehydrated in 100 µl PBS containing 60 µM Ni²⁺. Where indicated, as an alternative to LPS, either IFN-γ or GM-CSF (50 ng) was included in the rehydration buffer. Hydrated mixtures were sonicated (three times, 15 sec bursts) using a TOSCO 100W ultrasonic disintegrator (Measuring and Scientific Ltd., London, UK) at maximum amplitude. Liposomes (100 µl) were mixed with 100 µl of B16-OVA cell-derived PMV (1x10⁸ cell equivalents), before adding 15% PEG₄₀₀ and diluting 10 times with PBS. The (NTA)₃-DTDA- and cytokine-containing PMV were purified by size-exclusion chromatography,¹⁷ before engrafting with the appropriate ScFv.

Stealth Liposomes (SL) were prepared as follows: POPC, (NTA)₃-DTDA, PE-PEG₂₀₀₀, LPS and PC-BODIPY (molar ratio 96:1:1:1:1); or POPC, (NTA)₃-DTDA, PE-PEG₂₀₀₀ and PC-BODIPY (molar ratio 97:1:1:1) dissolved in ethanol were dried under a stream of N₂, then rehydrated in 100 µl PBS containing 30 µM Ni²⁺ (total lipid 1 mM). For mixtures lacking LPS, IFN-γ or GM-CSF (50 ng) was included in the PBS. Lipid mixtures were sonicated and SL purified (as above). For functional studies the PC-BODIPY was omitted from all lipid mixtures.

Encapsulation of the immunodominant epitope of the OVA protein, SIINFEKL, into SL was attempted but proved difficult since this peptide has low solubility at the pH used to produce the SL and to engraft histidine-tagged ScFv (pH 7.4). However, a hexahistidine-tagged form of the peptide, SIINFEKL-6H, permitted efficient encapsulation and/or engraftment of the peptide onto (NTA)₃-DTDA-containing SL. Binding studies using FACS analysis showed that CD11c-ScFv or DEC-205-ScFv-engrafted SL containing SIINFEKL-6H could effectively target receptors on DCs *in vitro* (not shown). Thus, where indicated, SIINFEKL-6H (2 µM) was included to simultaneously engraft with ScFv. The efficient encapsulation of OVA into SL containing POPC, (NTA)₃-DTDA and PE-PEG₂₀₀₀, was achieved by rehydrating the desiccated lipid mixture in PBS containing 0.1 mg OVA (1 mg/ml), followed by brief sonication. The (NTA)₃-DTDA-containing PMV and SL were engrafted by incubating with the appropriate ScFv (200 µg/ml) for 1 hr at room temperature. The binding of engrafted PMV and SL to DCs was assessed by flow cytometry as previously described.¹⁷

### Targeting of DC in vivo

In order to obtain highly fluorescent PMV for tracking studies PMV were reacted with fluorescein-isothiocyanate (FITC, Molecular Probes), engrafted with L2 or ScFv, and then injected into the hind footpad of mice. After 16 hrs the draining popliteal lymph node of each animal was harvested and used either for isolation of lymph node cells for two colour flow cytometric analysis after staining with biotinylated CD11c mAb and streptavidin-phycoerythrin (streptavidin-PE), or for confocal fluorescence imaging. For imaging, lymph nodes were fixed in 10% formalin, then embedded in paraffin, and cut into sections; the sections were then adhered onto slides and de-waxed. Slides were blocked by incubation with PBS plus 20% goat serum (PBS-goat serum) for 30 min at room temperature, before incubating with mAb N418 to CD11c in PBS-goat serum for 1 hr at room temperature. The slides were then washed extensively in water and stained with streptavidin-Rhodamine in PBS-goat serum. After further washing, the slides were analysed for fluorescein and Rhodamine fluorescence using a Radiance 2000 fluorescence confocal microscope (Bio-Rad, Richmond, California). Images were acquired by Kalman averaging of 30 successive laser scans, and processed using Bio-Rad Image software.

### Antigen presentation assays

DCs were incubated with modified PMV or SL at 37 °C in complete medium for 4 hrs, and then washed to remove unbound PMV or SL, γ-irradiated (5000 rad), and aliquoted in growth medium (2x10⁴ cells/200µl/well) into a 96-well flat-bottom plate. Syngeneic T cells were added (2 x 10⁴/well) and the cells co-cultured for 4 days, before assessing proliferation by measuring incorporation of [³H]-thymidine.¹⁴ The proportion of proliferating CD4⁺ and CD8⁺ T cells in Ag presentation assays was assessed by labelling the T cells with CFSE (5 µM) prior to co-culture with DCs as described.³³ After 4 days co-culture cells were washed, stained with anti-mouse CD4 (clone L3T4)-Cy-Chrome (10 µg/ml), and anti-mouse CD8 (clone Ly-2)-PE (10 µg/ml), and analysed for CFSE-, Cy-Chrome-, and PE-fluorescence by flow cytometry.

### Cytotoxicity assays

Ag-specific CTL assays were performed similar to those described.³⁴ Syngeneic C57BL6 mice were immunized intravenously (i.v.) with PBS (control), or ScFv-engrafted B16-OVA cell-derived PMV or SL bearing Ag (as indicated). At day 14 after immunization, spleens were removed and T lymphocytes (effector T cells) were isolated as above. The T cells were then suspended in complete growth medium and aliquoted into 24-well flat-bottom plates (ICN Biomedicals) at a concentration of 1x10⁵ cells/well and co-cultured with 1x10⁵ γ-irradiated (5000 rad) B16-OVA cells. After 5 days of co-culture, the cytolytic activity of the T cells was assessed in a standard ⁵¹Cr-release assay, as described.¹⁶

### Immunisation of animals and tumour challenge in vivo

Mice were immunized by three i.v. tail vein injections given weekly, with PBS (control), or either ScFv-engrafted B16-OVA cell-derived PMV (2x10⁵ cell equivalents), or SL (~0.16 µg total lipid) bearing associated Ag (~0.2 µg of OVA or ~0.8 ng of SIINFEKL-6H), each suspended in a 200 µl volume of PBS. Two weeks after the last injection, the mice were challenged by the i.v. injection of 3x10⁵ B16-OVA cells. At day 16 the lungs were removed and the number of tumour foci was counted visually under a dissection microscope. Alternatively, mice were immunised with ScFv-engrafted B16-OVA PMV 3, 6 and 9 days after i.v. injection of 1.5x10⁵ B16-OVA cells.

### Example 1

### Liposomes can be used to target tumour antigens to DC both in vitro and in vivo

Two types of liposome preparations were used to target tumour Ags to DCs (see Figure 1 below). The first entailed the use of a crude preparation of tumour cell-derived PMV modified by engraftment of ScFv targeting DC, and the second was a preparation of Ag-containing stealth liposomes also engrafted with DC targeting ScFv. Stealth liposomes (SLs) are synthetic lipid structures which have been sterically stabilised by the inclusion of lipids such as PE-PEG₂₀₀₀, and, by virtue of their ability to escape non-specific elimination by the reticulo-endothelial system, can remain in the blood circulation for days following their intravenous administration.¹⁴ The use of the chelator lipid NTA-DTDA to modify tumour cells and tumour cell-derived PMV for engraftment of T cell costimulatory molecules has been described.^{15,16} We have recently produced a novel lipid, (NTA)₃-DTDA (Fig. 1A), which is related to NTA-DTDA, but by achieving a higher local density of NTA headgroups, can permit a more stable anchoring of histidine-tagged proteins onto both PMV and onto SLs (not shown). Thus, liposome attachment, via (NTA)₃-DTDA, of histidine-tagged ScFv against DC markers such as CD11c and DEC-205 allows effective targeting of the liposomes to DCs (Fig. 1 B).

To determine whether liposomes prepared in this manner can be used to target tumour antigens to DCs, we first explored the ability of this system to target Ag to DCs *in vitro.* In this study we used the highly metastatic melanoma cell line, B16-OVA, as this line secretes low levels of OVA which can be used as a surrogate secreted tumour-specific Ag (Ref. 17), enabling OVA-specific immune responses to be assessed. The B16-OVA tumour line is largely resistant to OVA-specific CTLs unless high avidity CTLs are used.¹⁷ PMV (B16-OVA-derived) could be modified to contain incorporated (NTA)₃-DTDA by fusion with synthetic liposomes composed of POPC, (NTA)₃-DTDA, and PC-BODIPY (molar ratio 96:2:2). Also, (NTA)₃-DTDA-containing SLs were produced from an appropriate mixture of lipids: POPC, PE-PEG₂₀₀₀, (NTA)₃-DTDA, and PC-BODIPY (molar ratio 96:2:1:1). SLs preparations could be made to contain OVA, or the OVA CTL epitope, SIINFEKL. The (NTA)₃-DTDA-containing PMV and SLs were engrafted with either a control hexahistidine-containing molecule (L2 peptide) or a hexahistidine-tagged ScFv against either CD11c or DEC-205. Since the modified membranes also contain PC-BODIPY as a fluorescent tracer, their targeting to DCs can be assessed by flow cytometry.

Incubation of long term culture DC (LTC-DC) with control-modified PMV (PMV-L2) increased the fluorescence intensity of the cells slightly (~2-fold above background), but their fluorescence after incubation with PMV engrafted with CD11c-ScFv (PMV-CD11c), or with DEC-205-ScFv (PMV-DEC-205), was 4-8-fold greater than control cells (Fig. 2A). LTC-DC incubated with SL engrafted with CD11c-ScFv (SL-CD11c) and DEC-205-ScFv (SL-DEC-205), also exhibited significant increases in binding (3-6-fold) above control cells (SL-L2) (Fig. 2B). Similarly, the incubation of foetal skin DC (FSDC) that express CD11c, with PMV or SLs engrafted with CD11c-ScFv, resulted in a fluorescence increase substantially above that of control cells (not shown). The binding specificity of the engrafted PMV and SLs to DCs could be tested using blocking mAbs. Thus, pre-incubation of DCs with an isotype-matched control mAb did not significantly reduce binding of either CD11c-ScFv- or DEC-205-ScFv-engrafted PMV or SLs to DC, but their pre-incubation with either the anti-CD11c mAb N418 or the anti-DEC-205 mAb NLDC145, inhibited binding of the respective ScFv-engrafted SL or PMV by approx. 90% (not shown). This demonstrates that the binding is specific for the engrafted ScFv.

To establish that ScFv-engrafted PMV could target DCs *in vivo,* we injected mice subcutaneously into the hind footpad with fluorescein-labelled PMV engrafted with ScFv, and then examined cells isolated from the draining popliteal lymph node for fluorescein fluorescence by flow cytometry, or sections of the draining lymph node by confocal scanning laser microscopy, after PE staining each with a CD11c mAb as a DC marker. The results show that the injection of mice with L2-, CD11c-ScFv or DEC-205-ScFv-engrafted PMV results in a high level of CD11c-specific-fluorescence in a relatively small population (2-2.5%) of lymph node cells, thus identifying these as DCs, both by FACS analysis and fluorescence microscopy (Fig. 3A and B, panels i, iii and v). Importantly, of the CD11c-positive cells, a greater proportion of fluorescein-labelled cells was seen in the lymph node of mice injected with ScFv-engrafted PMV (~1.7%) (Fig 3A and B, panels iv and vi), compared to mice injected with L2-engrafted (control) PMV (0.4%) (Fig. 3A and B, corresponding panels i, and ii). The findings show that ScFv-engrafted PMV can target DCs *in vivo.*

**Table 1**

| Liposome and modified plasma membrane vesicle preparations | | | |
|---|---|---|---|
| **Liposome Type** | **Antigen** | **Targeting molecule** | **Abbreviation used** |
| Plasma membrane | B16 melanoma | Control L2 peptide^{c} | PMV-L2 |
| vesicle (PMV)^{a} | antigens + OVA | CD11c-ScFv | PMV-CD11c |
| | | DEC-205-ScFv | PMV-DEC-205 |
| Stealth liposome (SL) | OVA | Control L2 peptide | OVA-SL-L2 |
| | | CD11c-ScFv | OVA-SL-CD11c |
| | | DEC-205-ScFv | OVA-SL-DEC-205 |
| Stealth liposome (SL) | SIINFEKL^{b} | Control L2 peptide | SIINFEKL-SL-L2 |
| | (OVA peptide) | CD11c-ScFv | SIINFEKL-SL-CD11c |
| | | DEC-205-ScFv | SIINFEKL-SL-DEC-205 |

| | | | |
|---|---|---|---|
| ^{a}PMV derived from B16-OVA melanoma cell line. ^{b}SIINFEKL immunodominant class I MHC epitope with H-2^{b} haplotype. ^{c}Control hexahistidine-containing molecule for coupling to liposomes. | | | |

### Example 2

### Liposome-mediated targeting of tumour antigens to dendritic cells induces potent tumour-specific immunity both in vitro and in vivo

To determine whether Ag-bearing PMV and SL targeted to DCs can induce functional Ag presentation to T cells, we initially examined the ability of ScFv-engrafted PMV and SL to stimulate T cell proliferation in an Ag-presentation assay. Splenic DCs isolated from C57BL/6 mice were pulsed separately with B16-OVA-PMV, SL bearing SIINFEKL-6H, or SL bearing OVA, engrafted with either a control histidine-tagged peptide (L2) or with ScFv against CD11c and DEC-205. After the incubation, the cells were co-cultured with purified syngeneic T cells and then pulsed with [³H]-thymidine to assess the rate of T cell proliferation. Compared to control cultures, DCs exposed to PMV or SL (SIINFEKL-6H or OVA bearing) engrafted with CD11c-ScFv induced substantially higher levels of T cell proliferation. Even greater rates of proliferation were seen when the T cells were co-cultured with DC exposed to PMV or SL engrafted with a DEC-205 ScFv (Fig. 4A). Ag-bearing PMV and SL engrafted with ScFv, therefore, can effectively deliver Ag to DCs and stimulate T cell proliferation.

Interestingly, studies using CFSE-labelled T cells revealed that the ratio of proliferating CD4⁺ to CD8⁺ T cells was dependent on the Ag used. Thus, co-cultures of T cells with DCs which had been pulsed with DEC-205-engrafted PMV consisted of ~60% CD8⁺ T cells and ~40% CD4⁺ T cells (Fig. 4B). In contrast, co-cultures of T cells and DCs pulsed with DEC-205-engrafted SL bearing the OVA peptide SIINFEKL-6H, contained ~80% CD8⁺ T cells and ~20% CD4⁺ T cells, consistent with SIINFEKL being a CD8⁺ T cell epitope. Notably, T cells cultured with DCs pulsed with DEC-205-engrafted SL encapsulating intact OVA contained fewer proliferating CD8⁺ T cells (~70%) and a significantly higher proportion ~30% of CD4⁺ T cells compared with the SIINFEKL cultures (Fig. 4B), consistent with OVA containing both CD4⁺ and CD8⁺ T cell epitopes. The relative proportions of proliferating CD4⁺ and CD8⁺ T cells in co-cultures with DCs pulsed with CD11c-ScFv targeted Ags revealed a pattern similar to DC pulsed with DEC-205-ScFv targeted Ag (not shown).

Recent studies have demonstrated the importance of danger signals during Ag exposure and DC maturation^{9,10} in determining the type of immune response initiated by DCs. Although studies showed that liposomes can target Ag to DCs *in vitro* and induce T cell responses, previous *in vivo* studies suggest that for this approach to succeed *in vivo,* the co-delivery of danger signals to DCs is required. Thus, in order to deliver both Ag and inflammatory stimuli to DCs simultaneously, we produced Ag-bearing modified PMV and SL that contained incorporated LPS, IFN-γ, or GM-CSF. We found that up to 1% LPS could be included in the lipid mixture, and that PMV and SL could be made to incorporate the cytokines GM-CSF and IFN-γ with high efficiency, without significantly interfering with the ability of ScFv engrafted SL to target DCs *in vitro,* as assessed by binding studies using flow cytometry. Moreover, since GM-CSF induces the proliferation of FSDC in serum-free medium, and IFN-γ inhibits their proliferation in complete medium,¹⁷ FSDC proliferation assays were used to monitor cytokine entrapment in the SL with >85% of the GM-CSF and >75% of IFN-γ being found to be incorporated (not shown).

To determine whether DC-targeted PMV or Ag-containing SL could generate CTL responses *in vivo*, we immunised C57BL6 mice with preparations that either lacked or contained danger signals such as LPS, IFN-γ, or GM-CSF. We then isolated splenic T cells, restimulated the cells *in vitro* with γ-irradiated B16-OVA tumour cells, and assessed their cytolytic activity towards B16-OVA cells in a standard ⁵¹Cr-release assay. Representative lytic curves are shown in Fig. 5A, for animals that were immunised with various PMV preparations engrafted with the DEC-205-ScFv. Little CTL activity was detected when mice were pre-immunised with PMV engrafted with the L2 peptide or with DEC-205-ScFv in the absence of a danger signal (Fig. 5A). Incorporation of either LPS or IFN-γ in the DEC-205-ScFv-engrafted PMV, however, resulted in the induction of high levels of cytolytic activity, with 50% specific lysis of target cells still occurring at a 1:1 effector to target ratio (Fig. 5A). In contrast, GM-CSF was a much less effective inducer of CTL activity.

For ease of comparison, the cytolytic activity of the various PMV and SL immunisation conditions are presented at the 25:1 effector to target ratio in Fig. 5B. Maximum CTL activity was observed with splenocytes from mice immunised with PMV or SL (SIINFEKL or OVA bearing) containing IFN-γ or LPS as the danger molecule. CD11c-ScFv-engrafted PMV and SL were somewhat less immunogenic, with GM-CSF being generally a less effective danger signal than IFN-γ or LPS but, nevertheless, inducing significant CTL activity when associated with PMV, and OVA containing SL. Interestingly, cultures containing T cells from animals injected with ScFv-engrafted PMV or SL lacking an associated "danger" signal, gave near background levels of lysis (Fig. 5A and B).

### Example 3

### Liposome-based vaccines that target DC induce protective immunity against tumours

Mice immunised with the various B16-OVA preparations were examined for their ability to resist an i.v. challenge of B16-OVA tumour cells, with lung metastases being quantified 16 days following tumour cell injection. Compared to control mice, a much lower number of metastases was observed in mice immunised with PMV or OVA-bearing SL engrafted with ScFv and containing either LPS or IFN-γ (Fig. 6). If the PMV or OVA-bearing SL were not engrafted with a ScFv and did not contain LPS or IFN-γ little protection to tumour cell challenge was detected. In stark contrast, SIINFEKL containing SL were unable to protect mice against tumour challenge (Fig. 6B), despite some of the vaccine constructs inducing potent CTL activity (Fig. 5). These data are consistent with the B16-OVA melanoma being resistant to clearance by CD8⁺ CTLs (Ref. 14).

To explore the effect of vaccination on pre-existing tumours, we injected a group of 6 mice with DEC-205-ScFv-engrafted PMV containing IFN-γ at 3 days *after* challenge with 1.5x10⁵ B16-OVA tumour cells. Interestingly, vaccinated mice subsequently did not show any signs of tumour development, whereas a group of six control animals had to be euthanised at day 22 due to an increasing tumour burden in the lungs which contained an average of 250±37 tumour foci each.

The high proportion of proliferating CD4⁺ T cells seen in Ag presentation assays (Fig. 4B), raised the question of whether these cells, rather than CD8⁺ T cells, play a role in the anti-tumour responses observed. CD4⁺ T cells recently have been implicated in the clearance of B16-OVA melanoma lung metastases through a mechanism involving the eosinophil chemokine eotaxin.¹⁴ The possibility that eosinophils are involved in the anti-tumour response induced by targeting Ag to DCs was explored in studies in which we immunised eotaxin knockout mice with PMV-DEC-205-ScFv. The results show that whereas the cytolytic activity of T cells from normal and eotaxin knockout mice are essentially identical (Fig. 7A), eotaxin knockout mice immunised with PMV-DEC-205 exhibit a marked deficiency in their ability to inhibit tumour growth and metastasis (Fig. 7B).

### Example 4

### Enhancing immunity to an infectious agent by targeting its associated antigens to dendritic cells

In this example, we demonstrate that the invention can be used to target antigens of an infectious agent to DCs. BCG is a mycobacterium containing many of the antigens also present in the pathogen *Mycobacterium tuberculi* which is the cause of tuberculosis in humans. In the example to be described here, BCG mycobacteria were used instead of *Mycobacterium tuberculi.* BCG mycobacteria were grown in culture, heat-killed, and labelled [by reacting with a tracer 6-(fluoresein-5(and-6)-carboxamido)hexanoic acid succinimidyl ester] to allow tracking, before modifying to permit targeting to DCs. Thus, the heat-killed BCG was mixed with an appropriate amount of Ni-(NTA)₃-DTDA, and briefly sonicated to permit incorporation of the chelator lipid into the BCG membrane vesicles containing the BCG antigens. Incorporation of the Ni-(NTA)₃-DTDA into the BCG membranes then enabled engraftment of ScFv to either CD11c or DEC-205 to allow specific targeting to the CD11c and DEC-205 markers, respectively, on DCs.

The specific targeting is evident from the graphs comprising Figure 8. The fluorescence profiles show that only BCG preparations engrafted with a ScFv targeting murine DCs exhibit binding to the murine DC cell line JAWS-II. There is no binding of the control BCG preparations engrafted with the non-targeting control protein L2. This indicates that the modified PMVs and liposomes of the invention can be used to target antigens associated with BCG to DCs *in vitro.*

Further experiments were conducted to verify that BCG preparations containing engrafted DC-targeting ScFv also enhance the immune response to BCG antigens when used as vaccines in animals. C57/BL6 mice were vaccinated intravenously with the engrafted BCG preparations using essentially the same vaccination regime as in Example 1 above. After 2-4 weeks the mice were sacrificed, their spleens removed for isolation of T cells and to assay for BCG-specific interferon-γ production. The results of an Elispot assay of interferon-γ production were obtained by culturing the T cells isolated from the spleens of the mice in the presence of heat-killed BCG for a period of three days before assaying the cultures for interferon-γ-producing cells. The results of these experiments are presented in Figure 9.

It can be seen from Figure 9 that the spleens from mice vaccinated with BCG preparations that had been engrafted with either of the two ScFv targeting DCs, show a higher number of interferon-γ-producing T cells (i.e., Elispots) compared to those vaccinated with BCG preparations that had been engrafted with the control protein L2 (as indicated). Immunomodulatory factors (e.g., interferon-γ, IL-4, IL-10) also can be included with the targeted BCG membrane preparations in order to elicit the most appropriate type of immune response. The results thus show that as well as targeting antigens to DCs to enhance tumour immunity (as exemplified above), the modified PMVs and liposomes of the invention can also be used to target antigens from an infectious agent to DCs *in vivo,* to induce or enhance immunity to the agent.

The term "comprise" and variants of the term such as "comprises" or "comprising" are used herein to denote the inclusion of a stated integer or stated integers but not to exclude any other integer or any other integers, unless in the context or usage an exclusive interpretation of the term is required.

### References

1. Fong, L. & E. G. Engleman. Dendritic cells in cancer immunotherapy. Annu. Rev. Immunol. 18, 245 (2000).
2. Heiser, A. et al. Induction of polyclonal prostate cancer-specific CTL using dendritic cells transfected with amplified tumor RNA. J. Immunol. 166, 2953 (2001).
3. Gatza, E. & Okada, C.Y. Tumor cell lysate-pulsed dendritic cells are more effective than TCR Id protein vaccines for active immunotherapy of T cell lymphoma. J. Immunol. 169, 5227 (2002).
4. Timmerman, J. M. et al. Idiotype-pulsed dendritic cell vaccination for B-cell lymphoma: clinical and immune responses in 35 patients. Blood 99, 1517 (2002).
5. Marten, A., Renoth, S. & Schmidt-Wolf, LG. Increase of the stimulatory effect of dendritic cells by pulsing with apoptotic bodies transfected with the MHC class II gene. Mol. Immunol. 39, 395 (2002).
6. Inaba, K., Metlay, J.P., Crowley, M.T. & Steinman, R.M. Dendritic cells pulsed with protein antigens in vitro can prime antigen-specific, MHC-restricted T cells in situ. J. Exp. Med. 172, 631 (1990).
7. Wilson, H.L., Ni, K. & O'Neill, H.C. Identification of progenitor cells in long-term spleen stromal cultures that produce immature dendritic cells. P.N.A.S USA 9, 4784 (2000).
8. Guermonprez, P. et al. Antigen presentation and T cell stimulation by dendritic cells. Annu. Rev. Immunol. 20, 61 (2002).
9. Hawiger, D. et al. Dendritic cells induce peripheral T cell unresponsiveness under steady state conditions in vivo. J. Exp. Med. 194, 769 (2001).
10. Bonifaz, L. et al. Efficient targeting of protein antigen to the dendritic cell receptor DEC-205 in the steady state leads to antigen presentation on Major Histocompatibility Complex class I products and peripheral CD8+ T cell tolerance. J. Exp. Med. 196, 1627 (2002).
11. Ignatious, R. et al. Presentation of proteins encapsulated in sterically stabilized liposomes by dendritic cells initiates CD8+ T-cell responses in vivo. Blood 96, 3505 (2000).
12. Fukasawa, M. et al. Liposome oligomannose-coated with neoglycolipid, a new candidate for a safe adjuvant for induction of CD8+ cytotoxic T lymphocytes. FEBS Letters 41, 353 (1998).
13. Serre, K. et al. Efficient presentation of multivalent antigens targeted to various cell surface molecules of dendritic cells and surface Ig of antigen-specific B cells. J. Immunol. 161, 6059 (1998).
14. Papahadjopoulos, D., Allen, T. M. & Gabizon, A. Sterically stabilized liposomes:improvements in pharmacokinetics and antitumor therapeutic efficacy. P.N.A.S USA 88, 11460 (1991).
15. van Broekhoven, C. L.; Parish, C.R., Vassiliou, G. & Altin, J.G. Engrafting costimulator molecules onto tumor cell surfaces with chelator lipids: a potentially convenient approach in cancer vaccine development. J. Immunol. 164, 2433 (2000).
16. van Broekhoven, C. L. & Altin, J.G. A novel approach for modifying tumor cell-derived plasma membrane vesicles to contain encapsulated IL-2 and engrafted costimulatory molecules for use in tumor immunotherapy. Int. J. Cancer 98, 63 (2002).
17. Mattes, J. et al. Immunotherapy of cytotoxic T cell-resistant tumors by T helper 2 cells: an eotaxin and STAT6-dependent process. J. Exp. Med. 197, 387 (2003).
18. Matzinger, P. An innate sense of danger. Sem. Immunol. 10, 399 (1998).
19. Lutz, M. B., Assmann, C.U., Girolomoni, G. & Ricciardi-Castagnoli, P. Different cytokines regulate antigen uptake and presentation of a precursor dendritic cell line. Eur. J. Immunol. 26, 586 (1996).
20. Sallusto, F., Cella, M., Danieli, C. & Lanzavecchia, A. Dendritic cells use macropinocytosis and the mannose receptor to concentrate macromolecules in the major histocompatability complex class II compartment: downregulation by cytokines and bacterial products. J. Exp. Med. 182, 389 (1995).
21. Chang, C. H., Furue, M. & Tamaki, K. Selective regulation of ICAM-1 and major histocompatibility complex class I and II molecule expression on epidermal langerhans cells by some of the cytokines released by keratinocytes and T cells. Eur. J Immunol. 24, 2889 (1994).
22. Reis e Sousa, C., Stahl, P.D. & Austyn, J.M. Phagocytosis of antigens by Langerhans cells in vitro. J. Exp. Med. 178, 509 (1993).
23. Loike, J. D. et al. CD11c/CD18 on neutrophils recognizes a domain at the N-terminus of the A alpha chain of fibrinogen. P. N. A. S. USA 88, 1044 (1991).
24. Bilsland, C. A., Diamond, M.S. & Springer, T.A. The leukocyte integrin p150,95 (CD11c/CD18) as a receptor for iC3b: activation by a heterologous beta subunit and localization of a ligand recognition site to the I domain. J. Immunol. 152, 4582 (1994).
25. Jiang, W. et al. The receptor DCE-205 expressed by dendritic cells and thymic epithelium cells is involved in antigen processing. Nature 375, 151 (1995).
26. Mahnke, K. et al. The dendritic cell receptor for endocytosis, DEC-205, can recycle and enhance antigen presentation via major histocompatibility complex class 11-positive lysosomal compartments. J Cell. Biol. 151, 673 (2000).
27. Altin, J. G., White, F.A.J. & Easton. C.J. Synthesis of the chelator lipid nitrilotriacetic acid ditetradecylamine (NTA-DTDA) and its use with IAsys biosensor to study receptor-ligand interactions on model membranes. Biochim. Biophys. Acta. 1513, 131 (2001).
28. van Broekhoven, C. L. & J. G. Altin. A novel system for convenient detection of low-affinity receptor-ligand interactions: chelator-lipid liposomes engrafted with recombinant CD4 bind to cells expressing MHC class II. Immunol. Cell Biol. 79, 274 (2001).
29. O'Neill, H. C., Jonas, N., Wilson, H. & Ni, K. Immunotherapeutic potential of dendritic cells generated in long-term stroma-dependent cultures. Cancer Biother. Radiopharm. 14, 263 (1999).
30. Madea, T., Balakrishnan, K. & Mehdi, S.Q. A simple and rapid method for the preparation of plasma membranes. Biochim. Biophys. Acta 731, 115 (1983).
31. Vremec, D. et al. The surface phenotype of dendritic cells purified from mouse thymus and spleen: investigation of the CD8 expression by a subpopulation of dendritic cells. J. Exp. Med. 176, 47 (1992).
32. Greenfield, E. A. et al. B7.2 expressed by T cells does not induce CD28-mediated costimulatory activity but retains CTLA4 binding. J. Immunol. 158, 2025 (1997).
33. Lyons, A. B. & Parish, C.R. Determination of lymphocyte division by flow cytometry. J. Immunol. Methods. 171, 131 (1994).
34. Chen, L., P. et al. Tumor immunogenicity determines the effect of B7 costimulation on T cell-mediated tumor immunity. J. Exp. Med. 179, 523 (1994).

## Claims

1. A composition for modulating immunity by the *in vivo* targeting of an antigen to dendritic cells, the composition comprising:
a preparation of antigen-containing membrane vesicles or antigen-containing liposomes having on the surface thereof a plurality of metal chelating groups, wherein the metal chelating groups are the headgroups of amphiphilic molecules present within the phospholipids and/or lipids comprising the vesicles or liposomes; and
a ligand, selected from the group consisting of an antibody, an antibody fragment and a domain antibody, which can specifically bind a receptor on said dendritic cells, said ligand being linked to a said metal chelating group via a metal affinity tag on said ligand; wherein,
said antigen-containing vesicles or liposomes include an immunomodulatory factor selected from a bacterial lipopolysaccharide and interferon-γ.

2. The composition according to claim 1, wherein said antigen-containing membrane vesicles are selected from the group consisting of tumour-derived plasma membrane vesicles, lymphocyte-derived plasma membrane vesicles, leucocyte-derived plasma membrane vesicles, and membranous preparations of bacteria, protozoa, viruses or fungi.

3. The composition according to claim 1, wherein said antigen-containing liposomes are stealth liposomes.

4. The composition according to any one of claims 1 to 3, wherein said antigen of said antigen-containing membrane vesicles and antigen-containing liposomes is selected from the group consisting of proteins, glycoproteins, peptides, polysaccharides, and DNA encoding any of the foregoing.

5. The composition according to any one of claims 1 to 4, wherein the antigen of said antigen-containing membrane vesicles or liposomes comprises a plurality of different antigens.

6. The composition according to any one of claims 1 to 5, wherein said antibody fragment is a single chain antibody fragment.

7. The composition according to any one of claims 1 to 6, wherein said ligand is for a receptor selected from the group consisting of CD 11c, DEC-205 (CD205), DC-SIGN (CD209), CD206 and CD207.

8. The compoosition according to claim 7, wherein the ligand is CD11-ScFv or DEC-205-ScFv.

9. The composition according to any one of claims 1 to 8, wherein said metal-affinity tag on said ligand is hexahistidine.

10. The composition according to any one of claims 1 to 9, wherein said amphiphilic molecules are selected from the group consisting of nitrilotriacetic acid ditetradecylamine, tri(nitrilotriacetic acid) ditetradecylamine, or nitrilotriacetic acid phosphatidylethanolamine.

11. A composition comprising a preparation of antigen-containing membrane vesicles or antigen-containing liposomes having on the surface thereof a plurality of metal chelating groups, wherein the metal chelating groups are the headgroups of amphiphilic molecules present within the phospholipids and/or lipids comprising the vesicles or liposomes; and
a ligand selected from the group consisting of an antibody, an antibody fragment and a domain antibody which can specifically bind a receptor on said dendritic cells, said ligand being linked to a said metal chelating group via a metal affinity tag on said ligand, wherein
said antigen-containing vesicles or liposomes include an immunomodulatory factor selected from a bacterial lipopolysaccharide and interferon-γ.

12. A composition as claimed in claim 11 as further defined in any one of claims 2 to 10.

13. A composition as claimed in claim 11 or claim 12 wherein said composition is suitable for modulating immunity by the *in vivo* targeting of an antigen to dendritic cells.

14. A method for preparing a composition for modulating an immune response by the *in vivo* targeting of an antigen to dendritic cells, the process comprising the steps of:
i) preparing antigen-containing membrane vesicles or antigen-containing liposomes;
ii) modifying said antigen-containing membrane vesicles or antigen-containing liposomes by the incorporation of at least one immunomodulatory factor selected from a bacterial lipopolysaccaride and interferon-γ;
iii) further modifying said antigen-containing membrane vesicles or antigen-containing liposomes by the incorporation of amphiphilic molecules, wherein said amphiphilic molecules include as headgroups a metal chelator group which lies on the surface of said antigen-containing membrane vesicles or antigen-containing liposomes when incorporated therein; and
iv) contacting the product of step (iii) with a ligand, selected from the group consisting of an antibody, an antibody fragment and a domain antibody which can specifically bind a receptor on said dendritic cells, wherein said ligand includes a metal affinity tag for binding to said chelator group.

15. The method according to claim 14, wherein said antigen-containing membrane vesicles prepared in step (i), said antigen-containing liposomes prepared in step (i), said antigen of said antigen-containing membrane vesicles and antigen-containing liposomes, said amphiphilic molecules incorporated in step (iii), said ligand contacted with the product of step (iii) or said metal-affinity tag on said ligand is as defined in any one of claims 2 to 10.

16. Composition as defined in any one of claims 1 to 10 for modulating an immune response in a subject.

17. The composition according to claim 16, wherein said modulating of an immune response is for the prevention or treatment of transplant rejection or an autoimmune disease.

18. The composition according to claim 17, wherein said autoimmune disease is type I diabetes, rheumatoid arthritis, systemic lupus erythematosus or multiple sclerosis.

19. Composition as defined in any one of claims 1 to 10 for preventing or treating a tumour in a subject, wherein said antigen included in said antigen-containing membrane vesicles or antigen-containing liposomes is a tumour antigen.

20. The composition according to claim 19, wherein said tumour is a melanoma, or a cancer of the prostate, bowel, breast or lung.

21. Composition as defined in any one of claims 1 to 10 for preventing or treating an infection in a subject, wherein said antigen included in said antigen-containing membrane vesicles or antigen-containing liposomes is an antigen from an agent causing the infection.

22. The composition according to claim 21, wherein the causative agent of said infection is a bacterium, a mycobacterium, a virus, or a fungus.

23. The composition according to any one of claims 16 to 22, wherein said subject is a human subject.

## Patentansprüche

1. Zusammensetzung zur Immunmodulation durch das in-vivo-Targeting eines Antigens auf dendritische Zellen, wobei die Zusammensetzung umfasst:
ein Präparat von Antigen-haltigen Membranvesikeln oder Antigen-haltigen Liposomen, auf deren Oberfläche eine Mehrzahl von Metall chelatierenden Gruppen vorliegt, wobei die Metall chelatierenden Gruppen die Kopfgruppen von amphiphilen Molekülen sind, die in den Phospholipiden und/oder Lipiden vorliegen, welche die Vesikel oder Liposomen umfassen; und
einen Liganden, der ausgewählt ist aus der Gruppe bestehend aus einem Antikörper, einem Antikörper-Fragment und einem Domänen-Antikörper, der spezifisch einen Rezeptor auf den dendritischen Zellen binden kann, wobei der Ligand an eine besagte Metall chelatierende Gruppe über eine Metallaffinitäts-Markierung auf dem Liganden geknüpft ist; wobei
die Antigen-haltigen Vesikel oder Liposomen einen lmmunmodulationsfaktor einschließen, der aus bakteriellem Lipopolysaccharid und Interferon-γ ausgewählt ist.

2. Zusammensetzung nach Anspruch 1, bei der die Antigen-haltigen Membranvesikel ausgewählt sind aus der Gruppe bestehend aus von Tumoren abgeleiteten Plasma-Membranvesikeln, von Lymphozyten abgeleiteten Plasma-Membranvesikeln, von Leukozyten abgeleiteten Plasma-Membranvesikeln und Membranpräparaten von Bakterien, Protozoen, Viren oder Pilzen.

3. Zusammensetzung nach Anspruch 1, bei der die Antigen-haltigen Liposomen Stealth-Liposomen sind.

4. Zusammensetzung nach irgendeinem der Ansprüche 1 bis 3, bei der das Antigen der Antigen-haltigen Membranvesikel und Antigen-haltigen Liposomen ausgewählt ist aus der Gruppe bestehend aus Proteinen, Glycoproteinen, Peptiden, Polysacchariden und DNA, die irgendeines der Vorstehenden kodiert.

5. Zusammensetzung nach irgendeinem der Ansprüche 1 bis 4, bei der das Antigen der Antigen-haltigen Membranvesikel oder Liposomen eine Mehrzahl von verschiedenen Antigenen umfasst.

6. Zusammensetzung nach irgendeinem der Ansprüche 1 bis 5, bei der das Antikörper-Fragment ein Einzelketten-Antikörper-Fragment ist.

7. Zusammensetzung nach irgendeinem der Ansprüche 1 bis 6, bei der der Ligand für einen Rezeptor ist, der ausgewählt ist aus der Gruppe bestehend aus CD11c, DEC-205 (CD205), DC-SIGN (CD209), CD206 und CD207.

8. Zusammensetzung nach Anspruch 7, bei der der Ligand CD11-ScFv oder DEC-205-ScFv ist.

9. Zusammensetzung nach irgendeinem der Ansprüche 1 bis 8, bei der die Metallaffinitäts-Markierung auf dem Liganden Hexahistidin ist.

10. Zusammensetzung nach irgendeinem der Ansprüche 1 bis 9, bei der die amphiphilen Moleküle ausgewählt sind aus der Gruppe bestehend aus Nitrilotriessigsäureditetradecylamin, Tri(nitrilotriessigsäure)ditetradecylamin oder Nitrilotriessigsäurephosphatidylethanolamin.

11. Zusammensetzung, umfassend ein Präparat von Antigen-haltigen Membranvesikeln oder Antigen-haltigen Liposomen, auf deren Oberfläche eine Mehrzahl von Metall chelatierenden Gruppen vorliegt, wobei die Metall chelatierenden Gruppen die Kopfgruppen von amphiphilen Molekülen sind, die in den Phospholipiden und/oder Lipiden vorliegen, welche die Vesikel oder Liposomen umfassen; und
einen Liganden, der ausgewählt ist aus der Gruppe bestehend aus einem Antikörper, einem Antikörper-Fragment und einem Domänen-Antikörper, der spezifisch einen Rezeptor auf den dendritischen Zellen binden kann, wobei der Ligand über eine Metallaffinitäts-Markierung auf dem Liganden an eine besagte Metall chelatierende Gruppe geknüpft ist, wobei
die Antigen-haltigen Vesikel oder Liposomen einen Immunmodulationsfaktor einschließen, der aus bakteriellem Lipopolysaccharid und Interferon-γ ausgewählt ist.

12. Zusammensetzung nach Anspruch 11, wie weiter in irgendeinem der Ansprüche 2 bis 10 definiert.

13. Zusammensetzung nach Anspruch 11 oder Anspruch 12, bei der die Zusammensetzung zur Immunmodulation durch das in-vivo-Targeting eines Antigens auf dendritische Zellen geeignet ist.

14. Verfahren zur Herstellung einer Zusammensetzung zum Modulieren einer Immunantwort durch das in-vivo-Targeting eines Antigens auf dendritische Zellen, wobei das Verfahren die Schritte umfasst:
i) Herstellen von Antigen-haltigen Membranvesikeln oder Antigen-haltigen Liposomen;
ii) Modifizieren der Antigen-haltigen Membranvesikel oder Antigen-haltigen Liposomen durch Einverleibung mindestens eines Immunmodulationsfaktors, der aus bakteriellem Lipopolysaccharid und Interferon-γ ausgewählt ist;
iii) weiteres Modifizieren der Antigen-haltigen Membranvesikel oder Antigen-haltigen Liposomen durch Einverleibung von amphiphilen Molekülen, wobei die amphiphilen Moleküle als Kopfgruppen eine Metallchelator-Gruppe einschließen, die auf der Oberfläche der Antigen-haltigen Membranvesikel oder Antigen-haltigen Liposomen vorliegt, wenn sie diesen einverleibt ist; und
iv) In-Kontakt-Bringen des Produkts von Schritt (iii) mit einem Liganden, der ausgewählt ist aus der Gruppe bestehend aus einem Antikörper, einem Antikörper-Fragment und einem Domänen-Antikörper, der spezifisch einen Rezeptor auf den dendritischen Zellen binden kann, wobei der Ligand eine Metallaffinitäts-Markierung zum Binden an die Chelator-Gruppe einschließt.

15. Verfahren nach Anspruch 14, bei dem die Antigen-haltigen Membranvesikel, die in Schritt (i) hergestellt werden, die Antigen-haltigen Liposomen, die in Schritt (i) hergestellt werden, das Antigen der Antigen-haltigen Membranvesikel und Antigen-haltigen Liposomen, die amphiphilen Moleküle, die in Schritt (iii) einverleibt werden, der Ligand, der mit dem Produkt von Schritt (iii) in Kontakt gebracht wird, oder die Metall-Affinitätsmarkierung auf dem Liganden wie in irgendeinem der Ansprüche 2 bis 10 definiert ist.

16. Zusammensetzung wie in irgendeinem der Ansprüche 1 bis 10 definiert, zur Modulation einer Immunantwort in einem Patienten.

17. Zusammensetzung nach Anspruch 16, bei der das Modulieren einer Immunantwort für die Verhütung oder Behandlung von Transplantatabstoßung oder einer Autoimmunkrankheit ist.

18. Zusammensetzung nach Anspruch 17, bei der die Autoimmunkrankheit Diabetes Typ I, rheumatoide Arthritis, systemischer Lupus erythematodes oder Multiple Sklerose ist.

19. Zusammensetzung wie in irgendeinem der Ansprüche 1 bis 10 definiert zur Verhütung oder Behandlung eines Tumors in einem Patienten, wobei das Antigen, das in den Antigen-haltigen Membranvesikeln oder Antigen-haltigen Liposomen eingeschlossen ist, ein Tumor-Antigen ist.

20. Zusammensetzung nach Anspruch 19, bei der der Tumor ein Melanom oder ein Krebs der Prostata, des Darms, der Brust oder der Lunge ist.

21. Zusammensetzung wie in irgendeinem der Ansprüche 1 bis 10 definiert, zur Verhütung oder Behandlung einer Infektion in einem Patienten, wobei das Antigen, das in den Antigen-haltigen Membranvesikeln oder Antigen-haltigen Liposomen eingeschlossen ist, ein Antigen von einem die Infektion verursachenden Agens ist.

22. Zusammensetzung nach Anspruch 21, bei der das verursachende Agens der Infektion ein Bakterium, ein Mykobakterium, ein Virus oder ein Pilz ist.

23. Zusammensetzung nach irgendeinem der Ansprüche 16 bis 22, bei der der Patient ein Mensch ist.

## Revendications

1. Composition destinée à moduler l'immunité par le ciblage *in vivo* d'un antigène des cellules dendritiques, la composition comprenant :
➢ une préparation de vésicules membranaires contenant des antigènes ou de liposomes contenant des antigènes ayant à leur surface une pluralité de groupes chélateurs de métaux, les groupes chélateurs de métaux étant les groupes de tête de molécules amphiphiles présentes à l'intérieur des phospholipides et/ou lipides comprenant les vésicules ou liposomes ; et
➢ un ligand, choisi dans le groupe constitué d'un anticorps, d'un fragment d'anticorps et d'un anticorps simple domaine, qui peut spécifiquement se lier à un récepteur sur lesdites cellules dendritiques, ledit ligand étant lié à un dit groupe chélateur de métaux par le biais d'un marqueur d'affinité pour le métal sur ledit ligand ; dans laquelle,
lesdits liposomes ou vésicules contenant des antigènes comprennent un facteur immunomodulateur choisi parmi un lipopolysaccharide bactérien et l'interféron-γ.

2. Composition selon la revendication 1, dans laquelle lesdites vésicules membranaires contenant des antigènes sont choisies dans le groupe constitué des vésicules membranaires plasmiques dérivant de tumeurs, des vésicules membranaires plasmiques dérivant de lymphocytes, des vésicules membranaires plasmiques dérivant de leucocytes, et des préparations membranaires de bactéries, protozoaires, virus ou champignons.

3. Composition selon la revendication 1, dans laquelle lesdits liposomes contenant des antigènes sont des liposomes furtifs.

4. Composition selon l'une quelconque des revendications 1 à 3, dans laquelle ledit antigène desdites vésicules membranaires contenant des antigènes et desdits liposomes contenant des antigènes est choisi dans le groupe constitué des protéines, des glycoprotéines, des peptides, des polysaccharides, et de l'ADN codant pour l'un quelconque de ceux qui précèdent.

5. Composition selon l'une quelconque des revendications 1 à 4, dans laquelle l'antigène desdits vésicules ou liposomes membranaires contenant des antigènes comprend une pluralité d'antigènes différents.

6. Composition selon l'une quelconque des revendications 1 à 5, dans laquelle ledit fragment d'anticorps est un fragment d'anticorps simple chaîne.

7. Composition selon l'une quelconque des revendications 1 à 6, dans laquelle ledit ligand est destiné à un récepteur choisi dans le groupe constitué de CD11c, DEC-205 (CD205), DC-SIGN (CD209), CD206 et CD207.

8. Composition selon la revendication 7, dans laquelle le ligand est CD11-ScFv ou DEC-205-ScFv.

9. Composition selon l'une quelconque des revendications 1 à 8, dans laquelle ledit marqueur d'affinité pour le métal sur ledit ligand est l'hexahistidine.

10. Composition selon l'une quelconque des revendications 1 à 9, dans laquelle lesdites molécules amphiphiles sont choisies dans le groupe constitué par l'acide nitrilotriacétique ditétra-décylamine, l'acide tri(nitrilotriacétique) ditétra-décylamine, ou l'acide nitrilotriacétique phosphatidyléthanolamine.

11. Composition comprenant une préparation à base de vésicules membranaires contenant des antigènes ou de liposomes contenant des antigènes ayant à leur surface une pluralité de groupes chélateurs de métaux, les groupes chélateurs de métaux étant les groupes de tête de molécules amphiphiles présentes à l'intérieur des phospholipides et/ou lipides comprenant les vésicules ou liposomes ; et
un ligand choisi dans le groupe constitué d'un anticorps, d'un fragment d'anticorps et d'un anticorps simple domaine, qui peut spécifiquement se lier à un récepteur sur lesdites cellules dendritiques, ledit ligand étant lié au dit groupe chélateur de métaux par le biais d'un marqueur d'affinité pour le métal sur ledit ligand, dans laquelle
lesdits liposomes ou vésicules contenant des antigènes comprennent un facteur immunomodulateur choisi parmi un lipopolysaccharide bactérien et l'interféron-γ.

12. Composition selon la revendication 11 telle que davantage définie dans l'une quelconque des revendications 2 à 10.

13. Composition selon la revendication 11 ou la revendication 12, ladite composition étant appropriée pour moduler l'immunité par le ciblage *in vivo* d'un antigène des cellules dendritiques.

14. Méthode de préparation d'une composition destinée à moduler une réponse immunitaire par le ciblage *in vivo* d'un antigène des cellules dendritiques, la méthode comprenant les étapes consistant à :
i) préparer des vésicules membranaires contenant des antigènes ou des liposomes contenant des antigènes ;
ii) modifier lesdites vésicules membranaires contenant des antigènes ou lesdits liposomes contenant des antigènes en incorporant au moins un facteur immunomodulateur choisi parmi un lipopolysaccharide bactérien et l'interféron-γ ;
iii) modifier encore lesdites vésicules membranaires contenant des antigènes ou lesdits liposomes contenant des antigènes en incorporant des molécules amphiphiles, lesdites molécules amphiphiles comprenant comme groupes de tête un groupe chélateur de métaux qui se trouve à la surface desdites vésicules membranaires contenant des antigènes ou desdits liposomes contenant des antigènes lorsqu'il est incorporé dans ceux-ci ; et
iv) mettre en contact le produit de l'étape (iii) avec un ligand, choisi dans le groupe constitué d'un anticorps, d'un fragment d'anticorps et d'un anticorps simple domaine, qui peut spécifiquement se lier à un récepteur sur lesdites cellules dendritiques, ledit ligand comprenant un marqueur d'affinité pour le métal destiné à se lier au dit groupe chélateur.

15. Méthode selon la revendication 14, dans laquelle lesdites vésicules membranaires contenant des antigènes préparées dans l'étape (i), lesdits liposomes contenant des antigènes préparés dans l'étape (i), ledit antigène desdites vésicules membranaires contenant des antigènes ou desdits liposomes contenant des antigènes, lesdites molécules amphiphiles incorporées dans l'étape (iii), ledit ligand mis en contact avec le produit de l'étape (iii) ou ledit marqueur d'affinité pour le métal sur ledit ligand sont tels que définis dans l'une quelconque des revendications 2 à 10.

16. Composition telle que définie dans l'une quelconque des revendications 1 à 10 destinée à moduler une réponse immunitaire chez un sujet.

17. Composition selon la revendication 16, dans laquelle ladite modulation d'une réponse immunitaire est destinée à prévenir ou traiter un rejet d'un organe transplanté ou une maladie autoimmune.

18. Composition selon la revendication 17, dans laquelle ladite maladie autoimmune est un diabète de type I, la polyarthrite rhumatoïde, le lupus érythémateux disséminé ou la sclérose en plaques.

19. Composition telle que définie dans l'une quelconque des revendications 1 à 10 destinée à prévenir ou traiter une tumeur chez un sujet, dans laquelle ledit antigène compris dans lesdites vésicules membranaires contenant des antigènes ou lesdits liposomes contenant des antigènes est un antigène de tumeur.

20. Composition selon la revendication 19, dans laquelle ladite tumeur est un mélanome, ou un cancer de la prostate, de l'intestin, du sein ou des poumons.

21. Compositions telle que définie dans l'une quelconque des revendications 1 à 10 destinée à prévenir ou traiter une infection chez un sujet, dans laquelle ledit antigène compris dans lesdites vésicules membranaires contenant des antigènes ou lesdits liposomes contenant des antigènes est un antigène provenant d'un agent causant l'infection.

22. Composition selon la revendication 21, dans laquelle l'agent causant ladite infection est une bactérie, une mycobactérie, un virus, ou un champignon.

23. Composition selon l'une quelconque des revendications 16 à 22, dans laquelle ledit sujet est un sujet humain.
